# EUROPEAN PATENT APPLICATION

(11) **EP 1 873 159 A1**
(43) Date of publication of application: **02.01.2008**
(21) Application number: 06090114.7
(22) Date of filing: 21.06.2006
(51) Int. Cl.: C07D 513/08, A61K 31/529

(54) **Substituted sulphonamido-macrocycles as Tie2 inhibitors and salts thereof, pharmaceutical compositions comprising same, methods of preparing same and uses of same**

(71) Applicant: Bayer Schering Pharma Aktiengesellschaft, 13353 Berlin (DE)
(72) Inventor: Hartung, Ingo, 13469 Berlin (DE); Briem, Hans, 28279 Bremen (DE); Kettschau, Georg, 13187 Berlin (DE); Thierauch, Karl-Heinz, 14169 Berlin (DE); Lücking, Ulrich, 10245 Berlin (DE); Bömer, Ulf, 16548 Glienicke/Nordbahn (DE); Shäfer, Martina, 13187 Berlin (DE); Ince, Stuart, 10559 Berlin (DE)

(57) **Abstract**

The invention relates to substituted sulfonamido-macrocycles according to the general formula (I) : 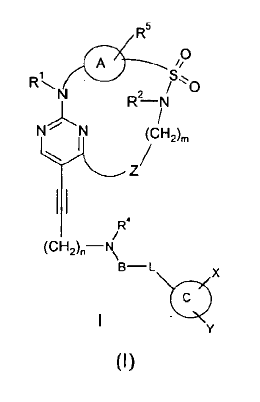 in which R¹, R², R⁴, R⁵, A, B, C, L, X, Y, Z, n, and m are as defined in the claims, and salts thereof, to pharmaceutical compositions comprising said substituted sulfonamido-macrocycles, to methods of preparing said substituted sulfonamido-macrocycles as well as the use thereof for manufacturing a pharmaceutical composition for the treatment of diseases of dysregulated vascular growth or of diseases which are accompanied with dysregulated vascular growth, wherein the compounds effectively interfere with Tie2 signalling.

## Description

The present invention relates to substituted sulfonamido-macrocycles of general formula (I) and salts thereof, to pharmaceutical compositions comprising said substituted sulfonamido-macrocycles, to methods of preparing said substituted sulfonamido-macrocycles, as well as to uses thereof.

Dysregulated vascular growth plays a critical role in a variety of inflammatory diseases, in particular psoriasis, delayed type hypersensitivity, contact dermatitis, asthma, multiple sclerosis, restenosis, rheumatoid arthritis and inflammatory bowl disease. Aberrant vascular growth is also involved in neovascular ocular diseases such as age-related macular degeneration and diabetic retinopathy. Additionally, sustained vascular growth is accepted as one hatlmark of cancer development (Hanahan, D.; Weinberg, R. A. Cell 2000, 100, 57). While tumours initially grow either as an avascular mass or by co-opting existing host vessels, growth beyond a few mm³ in size is depending on the induction of vessel neogrowth in order to sufficiently provide the tumour with oxygen and nutrients. Induction of angiogenesis is a prerequisite that the tumour surpasses a certain size (the so called angiogenic switch). An intricate signalling interaction network between cancer cells and the tumour microenvironment triggers the induction of vessel growth from existing vasculature. The dependence of tumours on neovascularization has led to a new treatment paradigm in cancer therapy (Ferrara et al. Nature 2005, 438, 967; Carmeliet Nature 2005, 438, 932). Blocking tumour neovascularization by small molecule or antibody-mediated inhibition of relevant signal transduction pathways holds a great promise for extending currently available therapy options.

The development of the cardiovascular system involves two basic stages. In the initial vasculogenesis stage, which only occurs during embryonal development, angioblasts differentiate into endothelial cells which subsequently form a primitive vessel network. The subsequent stage, termed angiogenesis, involves the remodelling of the initial vasculature and sprouting of new vessels (Risau, W. Nature 1997, 386, 671; Jain, R. K. Nat. Med. 2003, 9, 685). Physiologically, angiogenesis occurs in wound healing, muscle growth, the female cycle and in the above mentioned disease states.

It has been found that receptor tyrosine kinases of the vascular endothelial growth factor (VEGF) family and the Tie (tyrosine kinase with immunoglobulin and epidermal growth factor homology domain) receptor tyrosine kinases are essential for both developmental and disease-associated angiogenesis (Ferrara et al Nat. Med. 2003, 9, 669; Dumont et al. Genes Dev. 1994, 8, 1897; Sato et al. Nature 1995, 376, 70).

In adults the Tie2 receptor tyrosine kinase is selectively expressed on endothelial cells (EC) of the adult vasculature (Schlaeger et al. Proc. Nat. Acad. Sci. USA 1997, 94, 3058). Immunohistochemical analysis demonstrated the expression of Tie2 in adult rat tissues undergoing angiogenesis. During ovarian folliculogenesis, Tie2 is expressed in neovessels of the developing corpus luteum. Four endogeneous ligands - angiopoietins 1 to 4 - have been identified for the type 1 transmembrane Tie2 (also named Tek) receptor, while no ligands have been identified so far for the Tie1 receptor. Binding of the extracellular Tie2 domain to the C-terminal fibrinogen-like domains of the various angiopoietins leads to significantly different cellular effects. In addition, heterodimerizations between Tie1 and Tie2 receptors have been postulated to influence ligand binding.

Binding of Ang1 to Tie2 expressed on EC induces receptor cross-phosphorylation and kinase activation thus triggering various intracellular signalling pathways. The intracellular C-terminal tail of the Tie2 protein plays a crucial role in Tie2 signalling (Shewchuk et al. Structure 2000, 8, 1105). Upon ligand binding, a conformational change is induced which removes the C-tail out of its inhibitory conformation thus allowing kinase activation by cross-phoshorylation of various Tyr residues in the C-tail, which subsequently function as docking sites for phosphotyrosine-binding (PTB) site possessing down-stream mediators. Cellular effects initiated by Ang1 activation of Tie2 include inhibition of EC apoptosis, stimulation of EC migration and blood vessel reorganization, suppression of inflammatory gene expression and suppression of vascular permeability (Brindle et al. Circ. Res. 2006, 98, 1014). In contrast to VEGF-VEGFR signalling in EC, Ang1 activation of Tie2 does not stimulate EC proliferation in the majority of published assay settings.

The anti-apoptotic effect of Tie2 signalling was shown to be mediated mainly by the PI3K-Akt signalling axis which is activated by binding of the regulatory p85 subunit of PI3K to Y1102 in the Tie2 C-tail (DeBusk et al. Exp. Cell. Res. 2004, 298, 167; Papapetropoulos et al. J. Biol. Chem. 2000, 275, 9102; Kim et al. Circ. Res. 2000, 86, 24). In contrast, the chemotactic response downstream of the activated Tie2 receptor requires crosstalk between PI3K and the adaptor protein Dok-R. Membrane localization of Dok-R via binding of its plekstrin homology (PH) domain to PI3K and simultaneous binding to Y1108 in the Tie2 C-tail via its PTB domain leads to Dok-R phoshorylation and downstream signalling via Nck and Pak-1 (Jones et al. Mol. Cell Biol. 2003, 23, 2658; Master et al. EMBO J. 2001, 20, 5919). PI3K-mediated recruitment of the adaptor protein ShcA to Y1102 of the Tie2 C-tail is also believed to induce cellular sprouting and motility effects involving activation of endothelial nitric oxide synthase (eNOS), focal adhesion kinase (FAK) and the GTPases RhoA and Rac1. Other downstream mediators of Tie2 signalling include the adaptor protein Grb2, which mediates Erk1/2 stimulation, and the SHP-2 phosphatase.

In conclusion, basal activation of the Tie2 pathway by Ang1 is believed to maintain quiescence and integrity of the endothelium of the adult vasculature by providing a cell survival signal for ECs and by maintaining the integrity of the EC lining of blood vessels (Peters et al. Recent Prog. Horm. Res. 2004, 59, 51).

In contrast to Ang1, Ang2 is not able to activate Tie2 on EC unless Ang2 is present in high concentration or for prolonged periods. However, Ang2 functions as a Tie2 agonist in non-endothelial cells transfected with Tie2. The structural basis for this context-dependence of the Ang2-Tie2 interaction is to date not understood.

In endothelial cells, however, Ang2 functions as Tie2 antagonist and thus blocks the agonistic activity of Ang1 (Maisonpierre et al. Science 1997, 277, 55). Ang2 binding to Tie2 prevents Ang1-mediated Tie2 activation which leads to vessel destabilization and results in vessel regression in the absence of pro-angiogenic stimuli such as VEGF. While Ang1 is widely expressed by periendothelial cells in quiescent vasculature such as pericytes or smooth muscle cells, Ang2 expression occurs in areas of ongoing angiogenesis. Ang2 can be stored in Weibel-Palade bodies in the cytoplasm of EC allowing for a quick vascular response upon stimulation.

Ang1 and Ang2 are expressed in the corpus luteum, with Ang2 localizing to the leading edge of proliferating vessels and Ang1 localizing diffusively behind the leading edge. Ang2 expression is *inter alia* initiated by hypoxia (Pichiule et al. J. Biol. Chem. 2004, 279, 12171). Ang2 is upregulated in the tumour vasculature and represents one of the earliest tumour markers. In the hypoxic tumour tissue, Ang2 expression induces vessel permeability and - in the presence of e.g. pro-angiogenic VEGF - triggers angiogenesis. After VEGF mediated EC proliferation and vessel sprouting maturation of the newly formed vessels again necessitates Tie2 activation by Ang1. Therefore, a subtle balancing of Tie2 activity plays a pivotal role in the early as well as late stages of neovascularization. These observations render the Tie2 RTK an attractive target for anti-angiogenesis therapy in diseases caused by or associated with dysregulated vascular growth. However, it remains to be shown if targeting the Tie2 pathway alone will be sufficient to achieve efficacious blockade of neovascularization. In certain diseases or disease subtypes it might be necessary or more efficacious to block several angiogenesis-relevant signalling pathways simultaneously.

Various theories have been discussed to explain the differential effects of Ang1 and Ang2 on Tie2 downstream signalling events. Binding of Ang1 and Ang2 in a structurally different manner to the Tie2 ectodomain could induce ligand-specific conformational changes of the intracellular kinase domain explaining different cellular effects. Mutational studies however point toward similar binding sites of Ang1 and Ang2. In contrast, various publications have focussed on different oligomerization states of Ang1 vs. Ang2 as basis for different receptor multimerization states upon ligand binding. Only Ang1 present in its tetramer or higher-order structure initiates Tie2 activation in EC while Ang2 was reported to exist as a homodimer in its native state (Kim et al. J. Biol. Chem. 2005, 280, 20126; Davis et al. Nat. Struc. Biol. 2003, 10, 38; Barton et al. Structure 2005, 13, 825). Finally, specific interactions of Ang1 or Ang2 with additional cell-specific co-receptors could be responsible for the different cellular effects of Ang1 vs. Ang2 binding to Tie2. Interaction of Ang1 with integrin□α5ß1 has been reported to be essential for certain cellular effects (Carlson et al. J. Biol. Chem. 2001, 276, 26516; Dallabrida et al. Circ. Res. 2005, 96*,* e8). Integrin α5β1 associates constitutively with Tie2 and increases the receptor's binding affinity for Ang1 resulting in initiation of downstream signalling at lower Ang1 effector concentrations in situations where integrin□ α5β1 is present. The recently solved crystal structure of the Tie2-Ang2 complex suggests however that neither the oligomerization state nor a different binding mode causes the opposing cellular effects (Barton et al. Nat. Struc. Mol. Biol. 2006**,** advance online publication).

Ang1-Tie2 signalling plays also a role in the development of the lymphatic system and in lymphatic maintenance and sprouting (Tammela et al. Blood 2005, 105, 4642). An intimate cross-talk between Tie2 and VEGFR-3 signalling in lymphangiogenesis seems to equal the Tie2-KDR cross-talk in blood vessel angiogenesis.

A multitude of studies have underscored the functional significance of Tie2 signalling in the development and maintenance of the vasculature. Disruption of Tie2 function in Tie2^{-/-} transgenic mice leads to early embryonic lethality between days 9.5 and 12.5 as a consequence of vascular abnormalities. Tie2^{-/-} embryos fail to develop the normal vessel hierachy suggesting a failure of vascular branching and differentiation. The heart and vessels in Tie2^{-/-} embryos show a decreased lining of EC and a loosened interaction between EC and underlying pericyte/smooth muscle cell matrix. Mice lacking functional Ang1 expression and mice overexpressing Ang2 display a phenotype reminiscent of the phenotype of Tie2^{-/-} mice (Suri et al. Cell 1996, 87, 1171). Ang2^{-/-} mice have profound defects in the growth and patterning of lymphatic vasculature and fail to remodel and regress the hyaloid vasculature of the neonatal lens (Gale et al. Dev. Cell 2002, 3, 411). Ang1 rescued the lymphatic defects, but not the vascular remodelling defects. Therefore, Ang2 might function as a Tie2 antagonist in blood vasculature but as a Tie2 agonist in developing lymph vasculature suggesting redundant roles of Ang1 and Ang2 in lymphatic development.

Aberrant activation of the Tie2 pathway is involved in various pathological settings. Activating Tie2 mutations leading to increased ligand-dependent and ligand-independent Tie2 kinase activity cause inherited venous malformations (Vikkula et al. Cell 1996, 87, 1181). Increased Ang1 mRNA and protein levels as well as increased Tie2 activation have been reported in patients with pulmonary hypertension (PH). Increased pulmonary arterial pressure in PH patients results from increased coverage of pulmonary arterioles with smooth muscle cells (Sullivan et al. Proc. Natl. Acad. Sci. USA 2003, 100, 12331). In chronic inflammatory diseases, like in psoriasis, Tie2 and the ligands Ang1 and Ang2 are greatly upregulated in lesions, whereas a significant decrease in expression of Tie2 and ligands occur under anti-psoriatic treatment (Kuroda et al. J. Invest. Dermatol 2001, 116, 713). Direct association of pathogenesis of disease with Tie2 expression has been demonstrated recently in transgenic mice overexpressing Tie2 (Voskas et al. Am. J. Pathol. 2005, 166, 843). In these mice overexpression of Tie2 causes a psoriasis-like phenotype (such as epidermal thickening, rete ridges and lymphocyte infiltration).These skin abnormalities are resolved completely upon suppression of transgene expression, thereby illustrating a complete dependence on Tie2 signalling for disease maintenance and progression.

Tie2 expression was investigated in human breast cancer specimens and Tie2 expression was found in the vascular endothelium both in normal breast tissue as well as in tumour tissue. The proportion of Tie2-positive microvessels was increased in tumours as compared to normal breast tissue (Peters et al. Br. J. Canc. 1998, 77, 51). However, significant heterogeneity in endothelial Tie2 expression was observed in clinical specimen from a variety of human cancers (Fathers et al. Am. J. Path. 2005, 167, 1753). In contrast, Tie2 and angiopoietins were found to be highly expressed in the cytoplasm of human colorectal adenocarcinoma cells indicating at the potential presence of an autocrine/paracrine growth loop in certain cancers (Nakayama et al. World J. Gastroenterol. 2005, 11, 964). A similar autocrine/paracrine Ang1-Ang2-Tie2 loop was postulated for certain human gastric cancer cell lines (Wang et al. Biochem. Biophys. Res. Comm. 2005, 337, 386).

The relevance of the Ang1-Tie2 signalling axis was challenged with various biochemical techniques. Inhibition of Ang1 expression by an antisense RNA approach resulted in decreased xenograft tumour growth (Shim et al. Int. J. Canc. 2001, 94, 6 ; Shim et al. Exp. Cell Research 2002, 279, 299). However, other studies report that experimental overexpression of Ang1 in tumour models leads to decreased tumour growth (Hayes et al. Br. J. Canc. 2000, 83, 1154; Hawighorst et al. Am. J. Pathol. 2002, 160, 1381; Stoeltzing et al. Cancer Res. 2003, 63, 3370). The latter results can be rationalized by the ligand's ability to stabilize the endothelial lining of vessels rendering vessels less sensitive for angiogenic stimuli. Interference with the dynamics of Ang1-Tie2 signalling either by over-stimulation or by stimulus deprivation seemingly leads to similar phenotypes.

The pharmacological relevance of inhibiting Tie2 signalling was tested applying various non-small molecule approaches. A peptidic inhibitor of Ang1/2 binding to Tie2 was shown to inhibit Ang1-induced HUVEC migration and angiogenesis induction in an in vivo model (Tournaire et al. EMBO Rep. 2005, 5, 1). Corneal angiogenesis induced by tumour cell conditioned medium was inhibited by a recombinant soluble Tie2 receptor (sTie2) despite the presence of VEGF (Lin et al. J. Clin. Invest. 1997, 100, 2072; see also Singh et al. Biochem. Biophys. Res. Comm. 2005, 332, 194). Gene therapy by adenoviral vector delivered sTie2 was capable of reducing tumour growth rates of a murine mammary carcinoma and a murine melanoma and resulted in reduction of metastasis formation (Lin et al. Proc. Natl. Acad. Sci. USA 1998, 95, 8829). Similar effects were observed with related sTie2 constructs (Siemeister et al. Cancer Res. 1999, 59, 3185) and a Tek-Fc construct (Fathers et al. Am. J. Path. 2005, 167, 1753).

Adenovirus-delivered anti-Tie2 intrabodies were shown to inhibit growth of a human Kaposi's sarcoma and a human colon carcinoma upon peritumoural administration (Popkov et al. Cancer Res. 2005, 65, 972). Histopathological analysis revealed a marked decrease in vessel density in treated vs. control tumours. Phenotypic simultaneous knockout of KDR and Tie2 by an adenovirus delivered intradiabody resulted in significantly higher growth inhibition of a human melanoma xenograft model than KDR knockout alone (Jendreyko et al. Proc. Natl. Acad. Sci. USA 2005, 102, 8293). Similarly, the bispecific Tie2-KDR intradiabody was more active in an *in vitro* EC tube formation inhibition assay than the two monospecific intrabodies alone (Jendreyko et al. J. Biol. Chem. 2003, 278, 47812). Systematic treatment of tumour-bearing mice with Ang2-blocking antibodies and peptide-Fc fusion proteins led to tumour stasis and elimination of tumour burden in a subset of animals (Oliner et al. Cancer Cell 2004, 6, 507). For a recent report on an immunization approach, see Luo et al. Clin. Cancer Res. 2006, 12, 1813.

However, from the above studies using biochemical techniques to interfere with Tie2 signalling it is not clear, whether similar phenotypes will be observed with small molecule inhibitors of the Tie2 kinase activity. Small molecule inhibitors of kinases by definition block only those cellular effects which are mediated by the receptor's kinase activity and not those which might involve the kinase only as a co-receptor or scaffolding component in multi-enzyme complexes. So far, only a single study using a small molecule Tie2 inhibitor has been published (Scharpfenecker et al. J. Cell Sci. 2005, 118, 771). It remains to be shown that small molecule inhibitors of the Tie2 kinase will be as efficacious in inhibiting angiogenesis as e.g. ligand antibodies, soluble decoy receptors or receptor intrabodies. As discussed above, in certain settings inhibition of Tie2 signalling alone might not be sufficient to induce an adequate antiangiogenic effect. Simultaneous inhibition of several angiogenesis relevant signalling pathways could overcome such inadequacies. In conclusion, there is a great need for novel chemotypes for small mocule inhibitors of the Tie2 kinase. Fine tuning of additive anti-angiogenic activities as well as pharmacokinetic parameters such as e.g. solubility, membrane permeability, tissue distribution and metabolism will finally allow for chosing compounds of accurate profiles for various diseases caused by or associated with dysregulated vascular growth.

To date, a small number of therapeutic agents with antiangiogenic activity have been approved for cancer treatment. Avastin (Bevacizumab), a VEGF neutralizing antibody, blocks KDR and VEGFR1 signalling and has been approved for first-line treatment of metastatic colorectal cancer. The small molecule multi-targeted kinase inhibitor Nexavar (Sorafenib) inhibits *inter alia* members of the VEGFR family and has been approved for the treatment of advanced renal cell carcinoma. Sutent (Sunitinib), another multi-targeted kinase inhibitor with activity vs. VEGFR family members, has been approved by the FDA for treatment of patients with gastrointestinal stromal tumours (GIST) or advanced kidney tumours. Several other small molecule inhibitors of angiogenesis-relevant targets are in clinical and preclinical development.

AMG-386, an angiopoietin-targeting recombinant Fc fusion protein, is in phase I clinical development in patients with advanced solid tumours. Several multi-targeted small molecule inhibitors with activity against Tie2 are (or have been) in preclinical evaluation for cancer therapy, including ABT-869, GW697465A and A-422885.88 (BSF466895). The first and most recent compound, however, was reported to possess higher inhibitory activity against other kinase targets including non-angiogenesis kinases and oncogenic kinases. This agent is therefore not considered to be a purely antiangiogenic agent and its applicability to non-cancer diseases remains to be shown.

Pyrimidines and their derivatives have been frequently described as therapeutic agents for diverse diseases. Various recently published patent applications describe their use as inhibitors of protein kinases, frequently targeting cell cycle kinases, for example in W02001064654 and WO 2002096888 for use as CDK inhibitors, in WO 2003032997 for use as CDK and Aurora A kinase inhibitors, in WO 2003063794 for use as Syk kinase inhibitors, in WO 2003078404 for use as ZAP-70 and/or Syk or FAK kinase inhibitors, in WO 2004074244 for use as Plk inhibitors, in WO 2005026158 as ZAP-70 and/or Syk kinase inhibitors, and in WO2006021544 as Plk1 inhibitors.

More specifically, certain substituted pyrimidine derivatives have been disclosed as inhibitors of protein kinases involved in angiogenesis, such as VEGFR-2 (KDR) and/or Tie2 kinase, for example benzimidazole substituted 2,4-diaminopyrimidines (WO 2003074515) or bis-2,4-anilino-pyrimidines (WO 2003066601). W02006044823 described pyrimidine-substituted alkynes as Tie2 and/or Lck inhibitors.

Very recently, pyrimidine derivatives in which the pyrimidine constitutes a part of a macrocyclic ring system have been reported to be inhibitors of CDKs and/or VEGFRs (WO 2004026881), or of CDK2 and/or CDK5, respectively (WO 2004078682). 5-Alkinyl-2,4-diaminopyrimidine were described *inter alia* as Plk and CDK inhibitors in US20030171359, as PKCtheta inhibitors in US20060025433, and as compounds with NGF-like activity in US20040087789.

A problem in using substituted 2,4-diaminopyrimidines for the treatment of diseases associated with dysregulated vascular growth is their often poor selectivity for angiogenesis relevant kinases, which are primarily expressed on endothelial cells (EC), over kinases expressed in proliferating non-endothelial cells (e.g. cell cycle modulating kinases). By affecting not only the endothelium of aberrantly growing vasculature but also proliferating non endothelial cells, prolonged treatment with such compounds may cause side effects which would limit their pharmaceutical applicability. The patent applications cited above render diaminopyrimidines to be well known cell cycle kinase inhibitors, more particularly potent CDK and Plk inhibitors. However, it would be highly advantageous to have compounds at one's disposal which target one or more angiogenesis-relevant kinases while possessing modest or, even more advantageously, negligible activity against cell cycle modulating kinases such as CDK2 and Plk1. Such a pharmacological profile is highly desirable for treating diseases of dysregulated vascular growth or diseases which are accompanied with dysregulated vascular growth, in particular solid tumours and metastases thereof, and is even more desirable for treating non-oncological diseases of dysregulated vascular growth or non-oncological diseases which are accompanied with dysregulated vascular growth, such as retinopathy, other angiogenesis dependent diseases of the eye, in particular cornea transplant rejection or age-related macular degeneration, rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel, and diseases such as coronary and peripheral artery disease.

Therefore the aim of the present invention is to provide compounds, which are useful for the treatment of diseases resulting from or associated with dysregulated vascular growth. Selective inhibitors of one or multiple kinases, which are expressed on endothelial cells and play a role in the angiogenic sprouting, remodelling and/or maturation of new blood vessels, shall be provided. More particularly, potent dual inhibitors of Tie2 and KDR kinase shall be provided. Furthermore the prior art problems shall be overcome by providing compounds which do not inhibit CDK2 and Plk1 at compound concentrations of relevance for their Tie2 and KDR inhibitory activity.

The solution to the above-mentioned novel technical problem is achieved by providing compounds derived, in accordance with the present invention, from a class of substituted sulfonamido-macrocycles and salts thereof, methods of preparing substituted sulfonamido-macrocycles, a pharmaceutical composition containing said substituted sulfonamido-macrocycles, uses of said substituted sulfonamido-macrocycles and a method for treating diseases with said substituted sulfonamido-macrocycles, all in accordance with the description, as defined in the claims of the present application.

The present invention thus relates to compounds of general formula (I) : 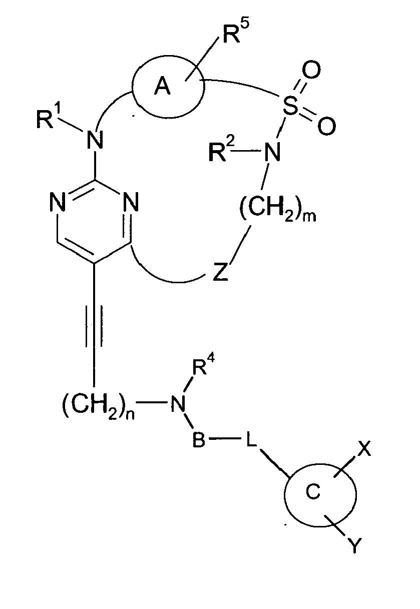 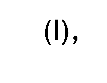 wherein
- A: is phenylene or C₆-heteroarylene;
- C: is arylene or heteroarylene ;
- Z: is selected from the group comprising, preferably consisting of, O, S, NR³ and CHR³ ;
- R¹, R², R³, R⁴ and R⁶: independently from each other are selected from the group comprising, preferably consisting of, hydrogen and C₁-C₆-alkyl, wherein C₁-C₆-alkyl is unsubstituted or singly or multiply substituted with -OR⁷ or -NR⁷R⁸;
- R⁵: is selected from the group comprising, preferably consisting of, hydrogen, halogen, nitro, cyano, C₁-C₆-alkyl, -(Ch₂)ₚOR^{7a}, - (CH₂)ₚNR^{7a}R^{8a}, -(CH₂)ₚC(O)R^{7a}, -(CH₂)ₚNHC(O)R^{7a}, - (CH₂)ₚNHC(O)NR^{7a}R^{8a}, -(CH₂)ₚNHS(O)₂R^{7a}, and -(CH₂)ₚC(O)NR^{7a}R^{8a} ;
- R⁷, R^{7a}, R^{7b}, R^{7c}, R^{7d}, R^{7e}, R⁸, R^{8a}, R^{8b}, R^{8c}, R^{8d}, and R^{8e}: independently from each other represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7b}, -NR^{7b}R^{8b}, C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a}; or
- R⁷ and R⁸, R^{7a} and R^{8a}, R^{7b} and R^{8b}, R^{7c} and R^{8c}, and R^{7e} and R^{8e}: together with the nitrogen atom to which they are attached in groups -NR⁷R⁸, -NR^{7a}R^{8a}, -NR^{7b}R^{8b}, -NR^{7c}R^{8c}, and -NR^{7e}R^{8e} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- R⁹, R^{9a},: independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
- R⁹ and R^{9a},: together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- B: is selected from the group comprising, preferably consisting of, -C(O)- , -C(S)-, -C(=NR⁶)-, -C(O)NR⁶-, -C(=NR⁶)NR⁶-, -S(O)₂-, -S(O)(=NR⁶)-, - S(=NR⁶)₂-, -C(S)NR⁶-, -C(O)C(O)-, -C(O)C(O)NR⁶-, -C(O)NR⁶C(O)-, - C(S)NR⁶C(O)-, -C(O)NR⁶C(S)-;
- L: represents a bond or is selected from the group comprising, preferably consisting of C₁-C₆-alkylene or C₃-C₁₀-cycloalkylene ;
- X: is selected from the group comprising, preferably consisting of, hydrogen, halogen, nitro, cyano, -(CH₂)ᵣOR^{7c}, -(CH₂)ᵣNR^{7c}R^{8c}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7c}, -(CH₂)ᵣC(O)NR^{7c}R^{8c} and - (CH₂)ᵣS(O)₂NR^{7c}R^{8c} ;
- Y: is selected from the group comprising, preferably consisting of, hydrogen, halogen, nitro, cyano, -(CH₂)ₛOR^{7e}, -(CH₂)ₛNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7e}, -(CH₂)ₛC(O)NR^{7e}R^{8e} and - (CH₂)ₛS(O)₂NR^{7e}R^{8e} ;
- m: represents an integer of 3, 4, 5, or 6 ;
- n: is an integer of 1 or 2 ; and
- p, q, q', r, s: independently from each other represent an integer of 0, 1, or 2.

In a preferred embodiment, the present invention relates to compounds of general formula (I) supra, wherein :
- A: is meta-phenylene ;
- C: is arylene or heteroarylene ;
- Z: is NR³ ;
- R¹, R², R³, and R⁵: are hydrogen ;
- R⁴ and R⁶: independently from each other, are selected from the group comprising, preferably consisting of, hydrogen and C₁-C₆-alkyl ;
- R^{7b}, R^{7c}, R^{7d}, R^{7e}, R^{8b}, R^{8c}, R^{8d}, and R^{8e}: independently from each other represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7b}, -NR^{7b}R^{8b}, C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a} ; or
- R^{7b} and R^{8b}, R^{7c} and R^{8c}, and R^{7e} and R^{8e}: together with the nitrogen atom to which they are attached in groups -NR^{7b}R^{8b}, -NR^{7c}R^{8c}, and -NR^{7e}R^{8e} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- R⁹, R^{9a},: independently from each other represent hydrogen, or are selected from the group comprising, preferably consisting of, C₁-C₆-alkyl, C₁-C₆-alkoxy, C₂-C₆-alkenyt, C₂-C₆-alkynyl, C₃-C₁₀-cycloalkyl, C₃-C₁₀-heterocycloalkyl, -(CH₂)_{q'}-aryl and -(CH₂)_{q},-heteroaryl, wherein said residues are unsubstituted or substituted one or more times independently from each other with halogen, nitro, cyano, C₁-C₆-alkyl, C₁-C₆-haloalkyl ; or
- R⁹ and R^{9a},: together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- B: is selected from the group comprising, preferably consisting of, -C(O)- , -C(S)-, -C(=NR⁶)-, -C(O)NR⁶-, -C(=NR⁶)NR⁶-, -S(O)₂-, -S(O)(=NR⁶)-, - S(=NR ⁶)₂-, -C(S)NR⁶-, -C(O)C(O)-, -C(O)C(O)NR⁶-, -C(O)NR⁶C(O)-, - C(S)NR⁶C(O)-, -C(O)NR⁶C(S)-;
- L: represents a bond or is selected from the group comprising, preferably consisting of C₁-C₆-alkylene or C₃-C₁₀-cycloalkylene ;
- X: is selected from the group comprising, preferably consisting of, hydrogen, halogen, nitro, cyano, -(CH₂)ᵣOR^{7c}, -(CH₂)ᵣNR^{7c}R^{8c}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7c}, -(CH₂)ᵣC(O)NR^{7c}R^{8c} and - (CH₂)ᵣS(O)₂NR^{7c}R^{8c} ;
- Y: is selected from the group comprising, preferably consisting of, hydrogen, halogen, nitro, cyano, -(CH₂)_{S}OR^{7e}, -(CH₂)ₛNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7e}, -(CH₂)ₛC(O)NR^{7e}R^{8e} and - (CH₂)ₛS(O)₂NR^{7e}R^{8e};
- m: represents an integer of 3 ;
- n: is an integer of 1 or 2 ; and
- q, q', r, s: independently from each other represent an integer of 0, 1, or 2.

In a more preferred embodiment, the present invention relates to compounds of general formula (I), supra, in which :
- A: is meta-phenylene ;
- C: is arylene or heteroarylene ;
- Z: is NR³ ;
- R¹, R² ,R³, and R⁵: are hydrogen ;
- R⁴ and R⁶: independently from each other, are selected from the group comprising, preferably consisting of, hydrogen and C₁-C₆-alkyl ;
- R^{7b}, R^{7c}, R^{7d} , R^{7e} , R^{8b}, R^{8c}, R^{8d}, and R^{8e}: independently from each other represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7b}, -NR^{7b}R^{8b} , C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a}; or
- R^{7b} and R^{8b}, R^{7c} and R^{8c}, and R^{7e} and R^{8e}: together with the nitrogen atom to which they are attached in groups -NR^{7b}R^{8b}, -NR^{7c}R^{8c}, and -NR^{7e}R^{8e} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- R⁹, R^{9a},: independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
- R⁹ and R^{9a},: together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
- B: is selected from the group comprising, preferably consisting of, -C(O)- , -C(O)NR⁶-, -S(O)₂- ;
- L: represents a bond or is selected from the group comprising, preferably consisting of C₁-C₆-alkylene or C₃-C₁₀-cycloalkylene ;
- X: is selected from the group comprising, preferably consisting of, hydrogen, halogen, nitro, cyano, -(CH₂)ᵣOR^{7c}, -(CH₂)ᵣNR^{7c}R^{8c}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7c}, -(CH₂)ᵣC(O)NR^{7c}R^{8c} and - (CH₂)ᵣS(O)₂NR^{7c}R^{8c} ;
- Y: is selected from the group comprising, preferably consisting of, hydrogen, halogen, nitro, cyano, -(CH₂)ₛOR^{7e}, -(CH₂)ₛNR^{7e}R^{8e}, C₁-C₆-alkyl, C₃-C₁₀-cycloalkyl, C₃-C₁₀-heterocycloakyl, C₁-C₆-haloalkyl, C₁-C₆-haloalkoxy, C₁-C₆-alkylthio, -(CH₂)ₛC(O)R^{7e}, -(CH₂)ₛC(O)NR^{7e}R^{8e} and - (CH₂)ₛS(O)₂NR^{7e}R^{8e};
- m: represents an integer of 3 ;
- n: is an integer of 1 or 2 ; and
- q, q', r, s: independently from each other represent an integer of 0, 1, or 2.

In a more particularly preferred embodiment, the present invention relates to compounds of general formula (I), supra, in which :
- A: is meta-phenylene ;
- C: is phenylene ;
- Z: is NR³ ;
- R¹, R², R³, R⁴, R⁵ and R⁶: are hydrogen ;
- R^{7b}, R^{7c}, R^{7d}, R^{7e}, R^{8b}, R^{8c},: independently from each other represent hydrogen, or C₁-C₆-alkyl, wherein said C₁-C₆-alkyl is unsubstituted or substituted one or more times independently from each other with -OR^{7b}, -NR^{7b}R^{8b} ; or
- R^{7b} and R^{8b}, R^{7c} and R^{8c},: together with the nitrogen atom to which they are attached in groups -NR^{7b}R^{8b}, and -NR^{7c}R^{8c}, form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen;
- B: is -C(O)NR⁶-, or -S(O)₂- ;
- L: represents a bond or is C₁-alkylene ;
- X: is selected from the group comprising, preferably consisting of, hydrogen, halogen, -(CH₂)ᵣOR^{7c}, -(CH₂)ᵣNR^{7c}R^{8c}, C₁-C₆-alkyl, C₁-C₆-haloalkyl, C₁-C₆-haloalkoxy;
- Y: is selected from the group comprising, preferably consisting of, hydrogen, halogen, -(CH₂)ₛOR^{7e}, C₁-C₆-alkyl, C₁-C₆-haloalkyl ;
- m: represents an integer of 3 ;
- n: is an integer of 1 or 2 ; and
- r: represents an integer of 0 or 1 ; and
- s: is an integer of 0.

In an even more particularly preferred embodiment, the present invention relates to compounds of general formula (I), supra, in which :
- A: is meta-phenylene ;
- C: is phenylene ;
- Z: is NR³ ;
- R¹, R², R³, R⁴, R⁵ and R⁶: are hydrogen ;
- R^{7b}, R^{7c}, R^{7d}, R^{7e}, R^{8b}, R^{8c},: independently from each other represent hydrogen, or C₁-C₆-alkyl, wherein said C₁-C₆-alkyl is unsubstituted or substituted one or more times independently from each other with -OR^{7b}, -NR^{7b}R^{8b}; or
- R^{7b} and R^{8b}, R^{7c} and R^{8c},: together with the nitrogen atom to which they are attached in groups -NR^{7b}R^{8b}, and -NR^{7c}R^{8c}, form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen ;
- B: is -C(O)NR⁶- ;
- L: represents a bond or is C₁-alkylene ;
- X: is selected from the group comprising, preferably consisting of, hydrogen, halogen, -(CH₂)ᵣOR^{7c}, -(CH₂)ᵣNR^{7c}R^{8c}, C₁-C₆-alkyl, C₁-C₆-haloalkyl, C₁-C₆-haloalkoxy ;
- Y: is selected from the group comprising, preferably consisting of, hydrogen, halogen, -(CH₂)ₛOR^{7e}, C₁-C₆-alkyl, C₁-C₆-haloalkyl ;
- m: represents an integer of 3 ;
- n: is an integer of 1 or 2 ; and
- r: represents an integer of 0 or 1 ; and
- s: is an integer of 0.

Even more particularly preferably still, the present invention relates to compounds of general formula (I), supra, in which :
- A: is meta-phenylene ;
- C: is phenylene ;
- Z: is NR³ ;
- R¹, R², R³, R⁴, R⁵ and R⁶: are hydrogen ;
- R^{7b} , R^{7c}, R^{7d}, R^{7e}, R^{8b}, R^{8c}: independently from each other represent hydrogen, or C₁-C₆-alkyl, wherein said C₁-C₆-alkyl is unsubstituted or substituted one or more times independently from each other with -OR^{7b}, -NR^{7b}R^{8b} ; or
- R^{7b} and R^{8b}, R^{7c} and R^{8c},: together with the nitrogen atom to which they are attached in groups -NR^{7b}R^{8b}, and -NR^{7c}R^{8c}, form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen ;
- B: is -S(O)₂- ;
- L: represents a bond or is C₁-alkylene ;
- X: is selected from the group comprising, preferably consisting of, hydrogen, halogen, -(CH₂)ᵣOR^{7c}, -(CH₂)ᵣNR^{7c}R^{8c}, C₁-C₆-alkyl, C₁-C₆-haloalkyl, C₁-C₆-haloalkoxy ;
- Y: is selected from the group comprising, preferably consisting of, hydrogen, halogen, -(CH₂)ₛOR^{7e}, C₁-C₆-alkyl, C₁-C₆-haloalkyl ;
- m: represents an integer of 3 ;
- n: is an integer of 1 or 2 ; and
- r: represents an integer of 0 or 1 ; and
- s: is an integer of 0.

The terms as mentioned herein and in the claims have preferably the following meanings :
The term "alkyl", as used in the context of the term "alkyl" or "alkylcarbonyl", for example, is to be understood as preferably meaning branched and unbranched alkyl, meaning e.g. methyl, ethyl, *n*-propyl, *iso*-propyl, *n*-butyl, *iso*-butyl, *tert-*butyl, *sec*-butyl, pentyl, *iso*-pentyl, hexyl, heptyl, octyl, nonyl and decyl and the isomers thereof.
The term "haloalkyl" is to be understood as preferably meaning branched and unbranched alkyl, as defined *supra,* in which one or more of the hydrogen substituents is replaced in the same way or differently with halogen. Particularly preferably, said haloalkyl is, e.g. chloromethyl, fluoropropyl, fluoromethyl, difluoromethyl, trichloromethyl, 2,2,2-trifluoroethyl, pentafluoroethyl, bromobutyl, trifluoromethyl, iodoethyl, and isomers thereof.
The term "alkoxy" is to be understood as preferably meaning branched and unbranched alkoxy, meaning *e.g.* methoxy, ethoxy, propyloxy, *iso*-propyloxy, butyloxy, iso-butyloxy, tert-butyloxy, sec-butyloxy, pentyloxy, *iso*-pentyloxy, hexyloxy, heptyloxy, octyloxy, nonyloxy, decyloxy, undecyloxy and dodecyloxy and the isomers thereof.
The term "alkylthio" is to be understood as preferably meaning branched and unbranched alkylthio, meaning *e.g.* methylthio, ethylthio, propylthio, *iso-*propylthio, butylthio, iso-butylthio, tert-butylthio, sec-butylthio, pentylthio, *iso-*pentylthio, hexylthio, heptylthio, octylthio, nonylthio, decylthio, undecylthio and dodecylthio and the isomers thereof.
The term "haloalkoxy" is to be understood as preferably meaning branched and unbranched alkoxy, as defined *supra,* in which one or more of the hydrogen substituents is replaced in the same way or differently with halogen, e.g. chloromethoxy, fluoromethoxy, pentafluoroethoxy, fluoropropyloxy, difluoromethyloxy, trichloromethoxy, 2,2,2-trifluoroethoxy, bromobutyloxy, trifluoromethoxy, iodoethoxy, and isomers thereof.
The term "cycloalkyl" is to be understood as preferably meaning a C₃-C₁₀ cycloalkyl group, more particularly a saturated cycloalkyl group of the indicated ring size, meaning e.g. a cyclopropyl, cyclobutyl, cyclopentyl, cyclohexyl, cycloheptyl, cyclooctyl, cyclononyl, or cyclodecyl group ; and also as meaning an unsaturated cycloalkyl group containing one or more double bonds in the C-backbone, e.g. a C₃-C₁₀ cycloalkenyl group, such as, for example, a cyclopropenyl, cyclobutenyl, cyclopentenyl, cyclohexenyl, cycloheptenyl, cyclooctenyl, cyclononenyl, or cyclodecenyl group, wherein the linkage of said cyclolalkyl group to the rest of the molecule can be provided to the double or single bond.
The term "heterocycloalkyl" is to be understood as preferably meaning a C₃-C₁₀ cycloalkyl group, as defined *supra,* featuring the indicated number of ring atoms, wherein one or more ring atoms are heteroatoms such as NH, NR^{8d}, oxygen or sulfur, or carbonyl groups, or, -otherwise stated - in a Cₙ-cycloalkyl group one or more carbon atoms are replaced by these heteroatoms to give such Cₙ cycloheteroalkyl group. Thus such group refers e.g. to a three-membered heterocycloalkyl, expressed as -C₃-heterocycloalkyl such as oxyranyl. Other examples of heterocycloalkyls are oxetanyl (C₄), aziridinyl (C₃), azetidinyl (C₄), tetrahydrofuranyl (C₅), pyrrolidinyl (C₅), morpholinyl (C₆), dithianyl (C₆), thiomorpholinyl (C₆), piperazinyl (C₆), trithianyl (C₆) and chinuclidinyl (C₈)-.
The term "halogen" or "Hal" is to be understood as preferably meaning fluorine, chlorine, bromine, or iodine.
The term "alkenyl" is to be understood as preferably meaning branched and unbranched alkenyl, e.g. a vinyl, propen-1-yl, propen-2-yl, but-1-en-1-yl, but-1-en-2-yl, but-2-en-1-yl, but-2-en-2-yl, but-1-en-3-yl, 2-methyl-prop-2-en-1-yl, or 2-methyl-prop-1-en-1-yl group.
The term "alkynyl" is to be understood as preferably meaning branched and unbranched alkynyl, e.g. an ethynyl, prop-1-yn-1-yl, but-1-yn-1-yl, but-2-yn-1-yl,or but-3-yn-1-yl group.

As used herein, the term "aryl" is defined in each case as having 3-12 carbon atoms, preferably 6-12 carbon atoms, such as, for example, cyclopropenyl, cyclopentadienyl, phenyl, tropyl, cyclooctadienyl, indenyl, naphthyl, azulenyl, biphenyl, fluorenyl, anthracenyl etc, phenyl being preferred.

As used herein, the term "heteroaryl" is understood as meaning an aromatic ring system which comprises 3-16 ring atoms, preferably 5 or 6 or 9 or 10 atoms, and which contains at least one heteroatom which may be identical or different, said heteroatom being such as oxygen, nitrogen or sulfur, and can be monocyclic, bicyclic, or tricyclic, and in addition in each case can be benzocondensed. Preferably, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl *etc.,* and benzo derivatives thereof, such as, e.g., benzofuranyl, benzothienyl, benzoxazolyl, benzimidazolyl, benzotriazolyl, indazolyl, indolyl, isoindolyl, *etc*.; or pyridyl, pyridazinyl, pyrimidinyl, pyrazinyl, triazinyl, *etc*., and benzo derivatives thereof, such as, for example, quinolinyl, isoquinolinyl, *etc.;* or azocinyl, indolizinyl, purinyl, *etc.,* and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, *etc.*

The term "alkylene", as used herein in the context of the compounds of general formula (I) is to be understood as meaning an optionally substituted alkyl chain or "tether", having 1, 2, 3, 4, 5, or 6 carbon atoms, i.e. an optionally substituted - CH₂- ("methylene" or "single membered tether" or e.g. -C(Me)₂-), -CH₂-CH₂-("ethylene", "dimethylene", or "two-membered tether"), -CH₂-CH₂-CH₂-("propylene", "trimethylene", or "three-membered tether"), -CH₂-CH₂-CH₂-CH₂-("butylene", "tetramethylene", or "four-membered tether"), -CH₂-CH₂-CH₂-CH₂-CH₂- ("pentylene", "pentamethylene" or "five-membered ether"), or -CH₂-CH₂-CH₂-CH₂-CH₂-CH₂- ("hexylene", "hexamethylene", or six-membered tether") group.

Preferably, said alkylene tether is 1, 2, 3, 4, or 5 carbon atoms, more preferably 1 or 2 carbon atoms.

The term "cycloalkylene", as used herein in the context of the compounds of general formula (I) is to be understood as meaning an optionally substituted cycloalkyl ring, having 3, 4, 5, 6, 7, 8, 9 or 10, preferably 3, 4, 5, or 6, carbon atoms, i.e. an optionally substituted cyclopropyl, cyclobutyl, cyclopentyl, cyclohexyl, cycloheptyl, cyclooctyl, cyclononyl, or cyclodecyl ring, preferably a cyclopropyl, cyclobutyl, cyclopentyl or cyclohexyl ring.

The term "heterocycloalkylene", as used herein in the context of the compounds of general formula (I) is to be understood as meaning a cycloalkylene ring, as defined *supra,* but which contains at least one heteroatom which may be identical or different, said heteroatom being such as oxygen, nitrogen or sulfur.

The term "phenylene", as used herein in the context of the compounds of general formula (I) which include the group A, is to be understood as meaning an optionally substituted all-carbon monocyclic six-membered arylene aromatic system or "tether". When the term "phenylene" is used it is to be understood that the linking residues can be arranged to each other in ortho-, para- or meta-position.

The term "arylene", as used herein in the context of the compounds of general formula (I) which include the groups B and C, is to be understood as meaning an optionally substituted monocyclic or polycyclic arylene aromatic system e.g. arylene, naphthylene and biarylene, preferably an optionally substituted phenyl ring or "tether", having 6 or 10 carbon atoms. More preferably, said arylene tether is a ring having 6 carbon atoms. When the term "arylene" is used it is to be understood that the linking residues can be arranged to each other in ortho-, para- or meta-position, e.g.an optionally substituted moiety of structure : 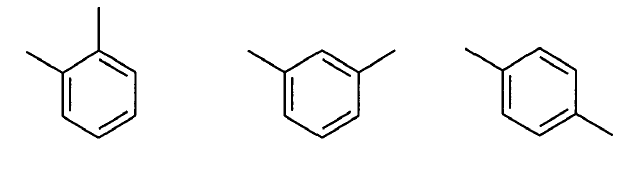 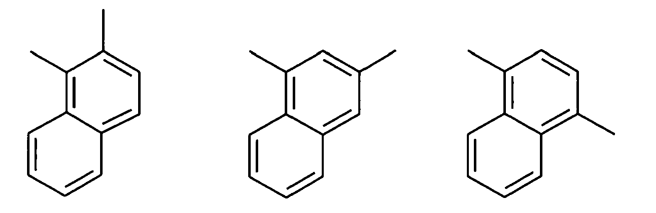 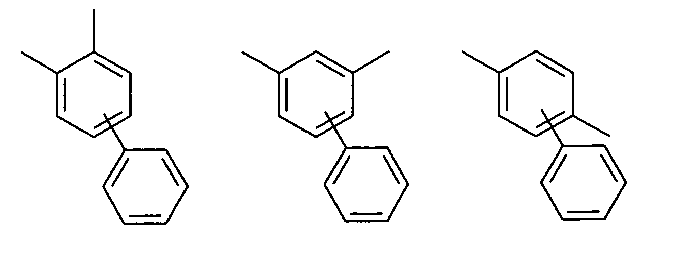 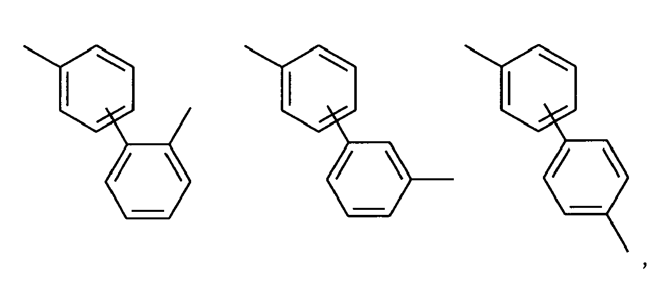 in which linking positions on the rings are shown as non-attached bonds.

The term "heteroarylene", as used herein in the context of the compounds of general formula (I) which include the groups A, B and C, is to be understood as meaning an optionally substituted monocyclic or polycyclic heteroarylene aromatic system, e.g. heteroarylene, benzoheteroarylene, preferably an optionally substituted 5-membered heterocycle, such as, for example, furan, pyrrole, thiazole, oxazole, isoxazole, or thiophene or "tether", or a 6-membered heterocycle, such as, for example, pyridine, pyrimidine, pyrazine, pyridazine. More preferably, said heteroarylene tether is a ring having 6 carbon atoms, e.g. an optionally substituted structure as shown *supra* for the arylene moieties, but which contains at least one heteroatom which may be identical or different, said heteroatom being such as oxygen, nitrogen or sulfur. When the term "heteroarylene" is used it is to be understood that the linking residues can be arranged to each other in ortho-, para- or meta-position.

As used herein, the term "C₁-C₆", as used throughout this text, e.g. in the context of the definition of "C₁-C₆-alkyl", or "C₁-C₆-alkoxy", is to be understood as meaning an alkyl group having a finite number of carbon atoms of 1 to 6, i.e. 1, 2, 3, 4, 5, or 6 carbon atoms. It is to be understood further that said term "C₁-C₆" is to be interpreted as any sub-range comprised therein, e.g. C₁-C₆, C₂-C₅, C₃-C₄, C₁-C₂, C₁-C₃, C₁-C₄, C₁-C₅ C₁-C₆ ; preferably C₁-C₂, C₁-C₃, C₁-C₄, C₁-C₅ , C₁-C₆ ; more preferably C₁-C₄.

Similarly, as used herein, the term "C₂-C₆", as used throughout this text, e.g. in the context of the definitions of "C₂-C₆-alkenyl" and "C₂-C₆-alkynyl", is to be understood as meaning an alkenyl group or an alkynyl group having a finite number of carbon atoms of 2 to 6, *i. e.* 2, 3, 4, 5, or 6 carbon atoms. It is to be understood further that said term "C₂-C₆" is to be interpreted as any sub-range comprised therein, e.g. C₂-C₆, C₃-C₅ , C₃-C₄, C₂-C₃, C₂-C₄, C₂-C₅ ; preferably C₂-C₃.

As used herein, the term "C₃-C₁₀", as used throughout this text, e.g. in the context of the definitions of "C₃-C₁₀-cycloalkyl", "C₃-C₁₀-heterocycloalkyl", or "C₃-C₁₀-cycloalkylene" is to be understood as meaning a cycloalkyl or heterocycloalkyl group or a cycloalkylene tether having a finite number of carbon atoms of 3 to 10, *i.e.* 3, 4, 5, 6, 7, 8, 9 or 10 carbon atoms, preferably 3, 4, 5 or 6 carbon atoms. It is to be understood further that said term "C₃-C₁₀" is to be interpreted as any sub-range comprised therein, e.g. C₃-C₁₀, C₄-C₉, C₅-C₈ , C₆-C₇; preferably C₃-C₆.

As used herein, the term "C₃-C₆", as used throughout this text, e.g. in the context of the definitions of "C₃-C₆-cycloalkyl", "C₃-C₆-heterocycloalkyl", or "C₃-C₆-cycloalkylene" is to be understood as meaning a cycloalkyl group or a heterocycloalkyl group or a cycloalkyl tether having a finite number of carbon atoms of 3 to 6, *i.e.* 3, 4, 5, or 6 carbon atoms. It is to be understood further that said term "C₃-C₆" is to be interpreted as any sub-range comprised therein, e.g. C₃-C₄, C₄-C₆, C₅-C₆.

Further, as used herein, the term "C₃-C₈", as used throughout this text e.g. in the context of the definitions of "C₃-C₈-cycloalkyl", is to be understood as meaning a cycloalkyl group having a finite number of carbon atoms of 3 to 8, i.e. 3, 4, 5, 6, 7 or 8 carbon atoms. It is to be understood further that said term "C₃-C₈" is to be interpreted as any sub-range comprised therein, e.g. C₃-C₈, C₄-C₇, C₅-C₆, C₃-C₄, C₃-C₅, C₃-C₆, C₃-C₇.

As used herein, the term "C₆-C₁₁", as used throughout this text, e.g. in the context of the definitions of "C₆-C₁₁-aryl", is to be understood as meaning an aryl group having a finite number of carbon atoms of 5 to 11, i.e. 5, 6, 7, 8, 9, 10 or 11 carbon atoms, preferably 5, 6, or 10 carbon atoms. It is to be understood further that said term "C₆-C₁₁" is to be interpreted as any sub-range comprised therein, e.g. C₅-C₁₀, C₆-C₉ , C₇-C₈; preferably C₅-C₆.

As used herein, the term "C₅-C₁₀", as used throughout this text, e.g. in the context of the definitions of "C₅-C₁₀-heteroaryl", is to be understood as meaning a heteroaryl group having a finite number of carbon atoms of 5 to 10, in addition to the one or more heteroatoms present in the ring i.e. 5, 6, 7, 8, 9, or 10 carbon atoms, preferably 5, 6, or 10 carbon atoms. It is to be understood further that said term "C₅-C₁₀" is to be interpreted as any sub-range comprised therein, *e.g.* C₆-C₉, C₇-C₈, C₇-C₈; preferably C₅-C₆.

As used herein, the term "C₁-C₃", as used throughout this text, *e.g.* in the context of the definitions of "C₁-C₃-alkylene", is to be understood as meaning an alkylene group as defined *supra* having a finite number of carbon atoms of 1 to 3, i.e. 1, 2, or 3. It is to be understood further that said term "C₁-C₃" is to be interpreted as any sub-range comprised therein, e.g. C₁-C₂, or C₂-C₃.

The term "isomers" is to be understood as meaning chemical compounds with the same number and types of atoms as another chemical species. There are two main classes of isomers, constitutional isomers and stereoisomers.

The term "constitutional isomers" is to be understood as meaning chemical compounds with the same number and types of atoms, but they are connected in differing sequences. There are functional isomers, structural isomers, tautomers or valence isomers.

In stereoisomers, the atoms are connected sequentially in the same way, such that condensed formulae for two isomeric molecules are identical. The isomers differ, however, in the way the atoms are arranged in space. There are two major subclasses of stereoisomers; conformational isomers, which interconvert through rotations around single bonds, and configurational isomers, which are not readily interconvertable.

Configurational isomers are, in turn, comprised of enantiomers and diastereomers. Enantiomers are stereoisomers which are related to each other as mirror images. Enantiomers can contain any number of stereogenic centers, as long as each center is the exact mirror image of the corresponding center in the other molecule. If one or more of these centers differs in configuration, the two molecules are no longer mirror images. Stereoisomers which are not enantiomers are called diastereomers. Diastereomers which still have a different constitution, are another sub-class of diastereomers, the best known of which are simple *cis - trans* isomers.

In order to limit different types of isomers from each other reference is made to IUPAC Rules Section E (Pure Appl Chem 45, 11-30, 1976).

The compound according to Fomula (I) can exist in free form or in a salt form. A suitably pharmaceutically acceptable salt of the substituted sulfonamido-macrocycles of the present invention may be, for example, an acid-addition salt of a substituted sulfonamido-macrocycle of the invention which is sufficiently basic, for example, an acid-addition salt with, for example, an inorganic or organic acid, for example hydrochloric, hydrobromic, sulfuric, phosphoric, trifluoroacetic, paratoluenesulfonic, methylsulfonic, citric, tartaric, succinic or maleic acid. In addition, another suitably pharmaceutically acceptable salt of a substituted sulfonamido-macrocycles of the invention which is sufficiently acidic is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, 1,6-hexadiamine, ethanolamine, glucosamine, sarcosine, serinol, tris-hydroxy-methyl-aminomethane, aminopropandiol, sovak-base, 1-amino-2,3,4-butantriol.

The compound according to Formula (I) can exist as N-oxides which are defined in that at least one nitrogen of the compounds of the general Formula (I) may be oxidized.

The compound according to Formula (I) can exist as solvates, in particular as hydrate, wherein the compound according to Formula (I) may contain polar solvents, in particular water, as structural element of the crystal lattice of the compounds. The amount of polar solvents, in particular water, may exist in a stoichiometric or unstoichiometric ratio. In case of stoichiometric solvates, e.g. hydrate, are possible hemi-, (semi-), mono-, sesqui-, di-, tri-, tetra-, penta- etc. solvates or hydrates, respectively.

As used herein, the term *"in vivo* hydrolysable ester" is understood as meaning an *in vivo* hydrolysable ester of a compound of formula (I) containing a carboxy or hydroxyl group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, *e.g.* methoxymethyl, C₁-C₆ alkanoyloxymethyl esters, *e.g.* pivaloyloxymethyl, phthalidyl esters, C₃-C₈ cycloalkoxy-carbonyloxy-C₁-C₆ alkyl esters, *e.g.* 1-cyclohexylcarbonyloxyethyl ; 1,3-dioxolen-2-onylmethyl esters, *e.g.* 5-methyl-1,3-dioxolen-2-onylmethyl ; and C₁-C₆-alkoxycarbonyloxyethyl esters, *e.g.* 1-methoxycarbonyloxyethyl, and may be formed at any carboxy group in the compounds of this invention. An *in vivo* hydrolysable ester of a compound of formula (I) containing a hydroxyl group includes inorganic esters such as phosphate esters and [alpha]-acyloxyalkyl ethers and related compounds which as a result of the *in vivo* hydrolysis of the ester breakdown to give the parent hydroxyl group.

Examples of [alpha]-acyloxyalkyl ethers include acetoxymethoxy and 2,2-dimethylpropionyloxymethoxy. A selection of *in vivo* hydrolysable ester forming groups for hydroxyl include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl.

Further another embodiment of the present invention relates to the use of a compound of general formula A, B as mentioned *infra* for the preparation of a compound of general formula (I) as defined *supra.*

The compounds of the present invention can be used in treating diseases of dysregulated vascular growth or diseases which are accompanied with dysregulated vascular growth. Especially, the compounds effectively interfere with Tie2 signalling. The compounds of the present invention show selective inhibition of Tie2 kinase and KDR kinase vs. e.g. CDK2 and Plk1 kinase. By primarily targeting endothelial cell-specific kinases compounds of the present invention may have an important advantage over prior art substances by reducing side effects which may result from interfering with signalling networks in non-endothelial cells. This effect can therefore allow prolonged treatment of patients with the compounds of the present invention offering good tolerability and high anti-angiogenic efficacy, where persistent angiogenesis plays a pathologic role.

Therefore, another aspect of the present invention is a use of the compound of general formula (I) described *supra* for manufacturing a pharmaceutical composition for the treatment of diseases of dysregulated vascular growth or of diseases which are accompanied with dysregulated vascular growth.

Preferably, the use is in the treatment of diseases, wherein the diseases are tumours and/or metastases thereof.

Another preferred use is in the treatment of diseases, wherein the diseases are retinopathy, other angiogenesis dependent diseases of the eye, in particular cornea transplant rejection or age-related macular degeneration, rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel.

A further use is in the treatment of diseases, wherein the diseases are coronary and peripheral artery disease.

Another use is in the treatment of diseases, wherein the diseases are ascites, oedema such as brain tumour associated oedema, high altitude trauma, hypoxia induced cerebral oedema, pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

Yet another aspect of the invention is a method of treating a disease of dysregulated vascular growth or diseases which are accompanied with dysregulated vascular growth, by administering an effective amount of a compound of general formula (I) described *supra.*

Preferably, the diseases of said method are tumours and/or metastases thereof.

Also, the diseases of said method are retinopathy, other angiogenesis dependent diseases of the eye, in particular cornea transplant rejection or age-related macular degeneration, e.g. rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel.

Further, the disease of the method is coronary and peripheral artery disease.

Other diseases of the method are ascites, oedema such as brain tumour associated oedema, high altitude trauma, hypoxia induced cerebral oedema, pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

The compounds of the present invention can thus be applied for the treatment of diseases accompanied by neoangiogenesis. This holds principally for all solid tumours, e.g. breast, colon, renal, lung and/or brain tumours or metastases thereof and can be extended to a broad range of diseases, where pathologic angiogenesis is persistent. This applies for diseases with inflammatory association, diseases associated with oedema of various forms and diseases associated with stromal proliferation and pathologic stromal reactions broadly. Particularly suited is the treatment for gynaecological diseases where inhibition of angiogenic, inflammatory and stromal processes with pathologic character can be inhibited. The treatment is therefore an addition to the existing armament to treat diseases associated with neoangiogenesis.

The compounds of the present invention can be used in particular in therapy and prevention of tumour growth and metastases, especially in solid tumours of all indications and stages with or without pre-treatment if the tumour growth is accompanied with persistent angiogenesis. However, this is not restricted to tumour therapy but is also of great value for the treatment of other diseases with dysregulated vascular growth. This includes retinopathy and other angiogenesis dependent diseases of the eye (e.g. cornea transplant rejection, age-related macular degeneration), rheumatoid arthritis, and other inflammatory diseases associated with angiogenesis such as psoriasis, delayed type hypersensitivity, contact dermatitis, asthma, multiple sclerosis, restenosis, pulmonary hypertension, stroke and inflammatory diseases of the bowel, such as Crohn's disease. This includes coronary and peripheral artery disease. This can be applied for disease states such as ascites, oedema, such as brain tumour associated oedema, high altitude trauma, hypoxia induced cerebral oedema, pulmonary oedema and macular oedema or oedema following burns and trauma. Furthermore, this is useful for chronic lung disease, adult respiratory distress syndrome. Also for bone resorption and for benign proliferating diseases such as myoma, benign prostate hyperplasia and wound healing for the reduction of scar formation. This is therapeutically valuable for the treatment of diseases, where deposition of fibrin or extracellular matrix is an issue and stroma proliferation is accelerated (e.g. fibrosis, cirrhosis, carpal tunnel syndrome etc). In addition this can be used for the reduction of scar formation during regeneration of damaged nerves, permitting the reconnection of axons. Further uses are endometriosis, pre-eclampsia, postmenopausal bleeding and ovarian hyperstimulation.

Another aspect of the present invention is a pharmaceutical composition which contains a compound of Formula (I) or pharmaceutically acceptable salts thereof, N-oxides, solvates, hydrates, isomers or mixtures of isomers thereof, in admixture with one or more suitable excipients. This composition is particularly suited for the treatment of diseases of dysregulated vascular growth or of diseases which are accompanied with dysregulated vascular growth as explained above.

In order that the compounds of the present invention be used as pharmaceutical products, the compounds or mixtures thereof may be provided in a pharmaceutical composition, which, as well as the compounds of the present invention for enteral, oral or parenteral application contain suitably pharmaceutically acceptable organic or inorganic inert base material, e.g. purified water, gelatin, gum Arabic, lactate, starch, magnesium stearate, talcum, vegetable oils, polyalkylenglycol, etc.

The pharmaceutical compositions of the present invention may be provided in a solid form, *e.g.* as tablets, dragées, suppositories, capsules or in liquid form, *e.g.* as a solution, suspension or emulsion. The pharmaceutical composition may additionally contain auxiliary substances, *e.g.* preservatives, stabilisers, wetting agents or emulsifiers, salts for adjusting the osmotic pressure or buffers.

For parenteral applications, (including intravenous, subcutaneous, intramuscular, intravascular or infusion), sterile injection solutions or suspensions are preferred, especially aqueous solutions of the compounds in polyhydroxyethoxy containing castor oil.

The pharmaceutical compositions of the present invention may further contain surface active agents, e.g. salts of gallenic acid, phosphorlipids of animal or vegetable origin, mixtures thereof and liposomes and parts thereof.

For oral application tablets, dragées or capsules with talcum and/or hydrocarbon-containing carriers and binders, e.g. lactose, maize and potato starch, are preferred. Further application in liquid form is possible, for example as juice, which contains sweetener if necessary.

The dosage will necessarily be varied depending upon the route of administration, age, weight of the patient, the kind and severity of the illness being treated and similar factors. The daily dose is in the range of 0.5 to 1,500 mg. A dose can be administered as unit dose or in part thereof and distributed over the day. Accordingly the optimum dosage may be determined by the practitioner who is treating any particular patient.

It is possible for compounds of general formula(I) of the present invention to be used alone or, indeed in combination with one or more further drugs, particularly anti-cancer drugs or compositions thereof. Particularly, it is possible for said combination to be a single pharmaceutical composition entity, e.g. a single pharmaceutical formulation containing one or more compounds according to general formula(I) together with one or more further drugs, particularly anti-cancer drugs, or in a form, e.g. a "kit of parts", which comprises, for example, a first distinct part which contains one or more compounds according to general formula I, and one or more further distinct parts each containing one or more further drugs, particularly anti-cancer drugs. More particularly, said first distinct part may be used concomitantly with said one or more further distinct parts, or sequentially.

Another aspect of the present invention is a method which may be used for preparing the compounds according to the present invention.

The following Table lists the abbreviations used in this paragraph and in the Examples section as far as they are not explained within the text body.

| Abbreviation | Meaning |
|---|---|
| Ac | Acetyl |
| Boc | *tert*-butyloxycarbonyl |
| br | Broad |
| c- | cyclo- |
| Cl | chemical ionisation |
| d | Doublet |
| dd | doublet of doublet |
| DCM | dichloromethane |
| DIPEA | N,N-diisopropylethyl amine |
| DMAP | N,N-dimethylaminopyridine |
| DMF | N,N-dimethylformamide |
| DMSO | dimethyl sulfoxide |
| eq. | Equivalent |
| ESI | electrospray ionisation |
| GP | general procedure |
| HPLC | high performance liquid chromatography |
| LC-MS | liquid chromatography mass spectrometry |
| m | Multiplet |
| mc | centred multiplet |
| MS | mass spectrometry |
| MTBE | methyl-tert-butyl ether |
| NMR | nuclear magnetic resonance spectroscopy : chemical shifts (δ) are given in ppm. |
| Pg | protecting group |
| q | quartet |
| rf | at reflux |
| r.t. or rt | room temperature |
| s | singlet |
| sept. | Septet |
| t | Triplet |
| T3P | 1-propanephosphonic acid cyclic anhydride |
| TEA | Triethylamine |
| TFA | trifluoroacetic acid |
| THF | tetrahydrofuran |
| TLC | Thin-layer chromatography |

NMR peak forms are stated as they appear in the spectra, possible higher order effects have not been considered. Chemical names were generated using AutoNom2000 as implemented in MDL ISIS Draw.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallisation. In some cases, impurities may be stirred out using a suitable solvent. In some cases, the compounds may be purified by chromatography, particularly flash column chromatography, using for example prepacked silica gel cartridges, *e.g.* from Separtis such as Isolute^{®} Flash silica gel or Isolute^{®} Flash NH₂ silica gel in combination with a Flashmaster II autopurifier (Argonaut/Biotage) and eluants such as gradients of hexane/EtOAc or DCM/ethanol. In some cases, the compounds may be purified by preparative HPLC using for example a Waters autopurifier equipped with a diode array detector and/or on-line electrospray ionization mass spectrometer in combination with a suitable prepacked reverse phase column and eluants such as gradients of water and acetonitrile which may contain additives such as trifluoroacetic acid or aqueous ammonia.

The following schemes and general procedures illustrate general synthetic routes to the compounds of general formula I of the invention and are not intended to be limiting. Specific examples are described in the subsequent paragraphs.

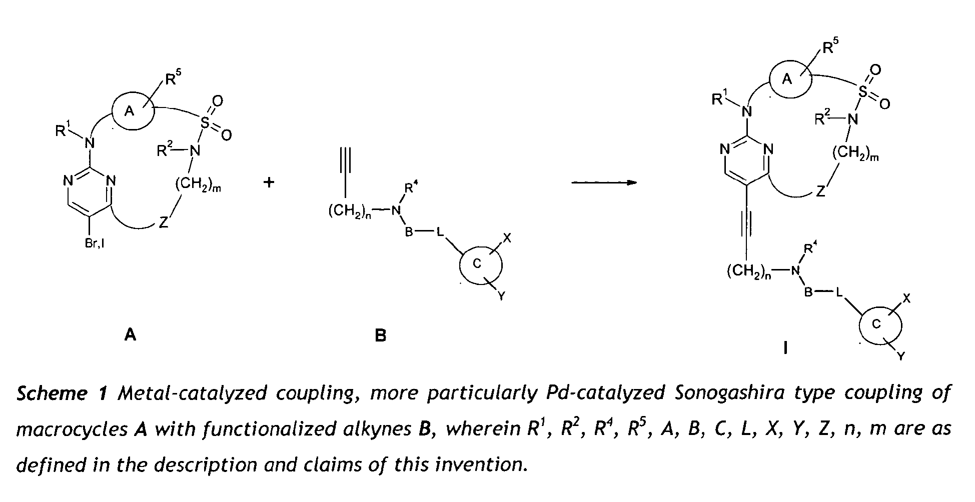

The compounds of the present invention can be prepared starting from halogenated macrocycles **(A)** by metal-catalyzed coupling reactions with respectively substituted alkynes **(B, Scheme 1)**. More particularly, compounds of the present invention can be prepared starting from brominated or iodinated macrocycles **(A)** by Pd-catalyzed Sonogashira-type coupling reactions with alkynes **(B)**. Transition metal-catalyzed couplings of heteroaryl bromides and iodides with alkynes are well known to the person skilled in the art (see for example Negishi, E.-i., Anastasia, L. Chem. Rev. 2003, 103, 1979; see also: Eur. J. Org. Chem. 2005, 20, 4256; J. Org. Chem. 2006, 71, 2535 and references therein; Chem. Commun. 2004, 17, 1934). In the so called Sonogashira coupling, reaction of terminal alkynes with (hetero)aryl halides is triggered by catalytic amounts of a Pd salt in the presence of a copper salt and a base. Various other Pd-catalyst/co-catalyst/ligand/base/solvent combinations have been published in the scientific literature which allow a fine-tuning of the required reaction conditions in order to allow for a broad set of additional functional groups on both coupling partners (see references in the above cited review). Additionally, recently developed procedures employing e.g. zinc acetylides, alkinyl magnesium salts or alkinyl trifluoroborate salts further broaden the scope of this process.

The synthesis of the required halogenated macrocycles **(A)** is described in WO 2004026881. The syntheses of two macrocycles of the formula A, in which R¹, R², R³ and R⁵ are standing for hydrogen, A is a meta-connected phenylene, Z is NH and m is the integer of 3, are herein exemplified as ***Preparation Example A,*** particularly with respect to the brominated macrocycle **A**, and even more particularly as ***Preparation Example B***, with respect to the iodinated macrocycle **A**. In addition to said two macrocycles, WO2004026881 specifically discloses a variety of procedures suitable for the preparation of various intermediates of the formula A, featuring various m, R⁵, R³, Z and A groups. These compounds may in turn be used for further modifications such as interconversions of R¹, R², and R³ groups and more particularly interconversions of R⁵ groups. For example, macrocyclic compounds of general formula **A**, wherein R⁵ stands for an amino group, may be further derivatized by standard amine transformations as known to the person skilled in the art, including, but not limited to, e.g. alkylations, acylations, sulfonylations, or urea formations.

The substituents R¹, R², R³, R⁴, R⁵, R⁶, X and Y in compounds of general formula (I) and in compounds of general formula A, B, B', B", B"', C, C', C" and C"' may be further modified.These modifications can be such as e.g. the introduction of protecting groups, cleavage of protecting groups, reduction or oxidation of functional groups, alkylations, substitution or other reactions. Appropiate protecting groups and their introduction and cleavage are well-known to the person skilled in the art (see for example T.W. Greene and P.G.M. Wuts in Protective Groups in Organic Synthesis, 3rd edition, Wiley 1999).

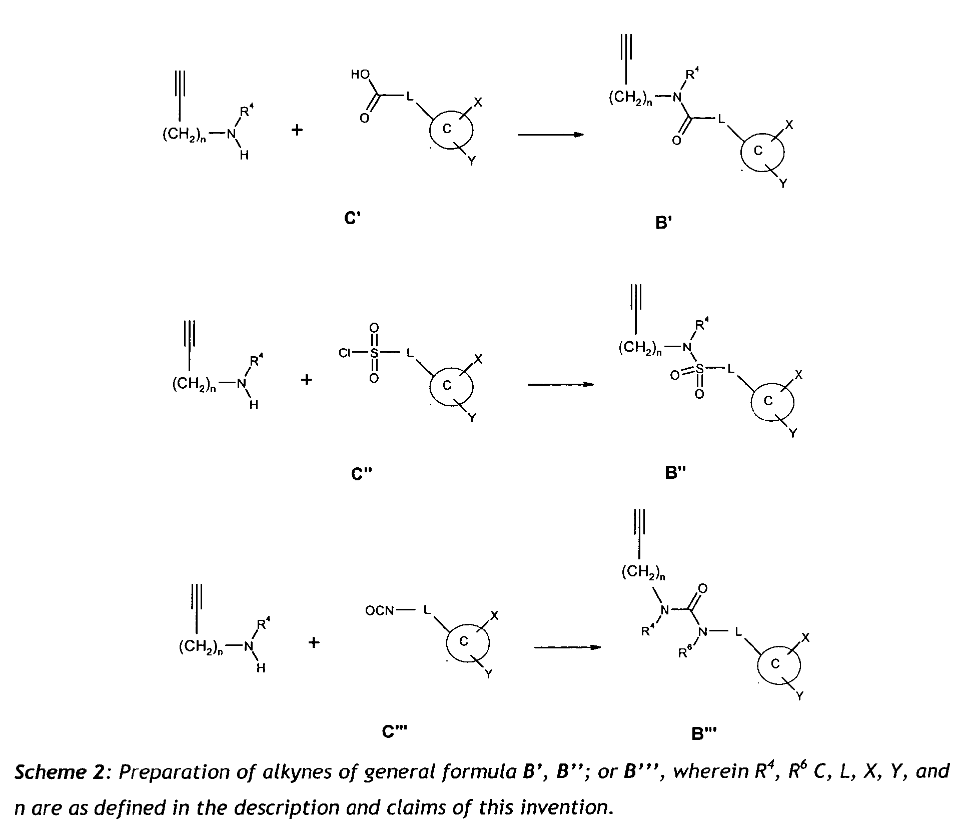

The appropriately substituted alkynes **(B)**, as needed for the above mentioned coupling reactions, can be prepared starting e.g. from propargyl amine or homopropargyl amine by standard transformations known to the person skilled in the art **(Scheme 2).** In particular, amides of general formula B' are accessible by reaction of e.g. propargyl amine or homopropargyl amine with carboxylic acids of general formula **C'**. Many methods for such amide formations are extractable from the scientific literature for the person skilled in the art including, but not limited to, pre-formation of the more reactive carboxylic acid chloride (by reaction of carboxylic acids with e.g. thionyl chloride or sulfuryl chloride or oxalyl chloride), or *in situ* activation of the carboxylic acid in the presence of the amine by reaction with coupling reagents e.g. dicyclohexylcarbodiimide (DCC)/dimethylaminopyridine (DMAP), ethyldimethylaminopropylcarbodiimide (EDC)/DMAP, N,N'-carbonyldiimidazole (CDI), or T3P and others known to the person skilled in the art. Peptide coupling conditions may be amenable as well. Sulfonamides of general formula **B"** are accessible by reaction of e.g. propargyl amine or homopropargyl amine with sulfonyl chlorides of general formula **C"**. Finally, ureas of general formula **B'"** are accessible by reaction of e.g. propargyl amine or homopropargyl amine with isocyanates of general formula **C"'**. The respective isocyanates are either commercially available or can be prepared from the respective amines by standard chemistry known to the person skilled in the art, particularly by reaction with phosgene equivalents.

The person skilled in the art is well aware of alternative methods of forming ureas, which may be of special importance in cases were the respective isocyanates are not readily available.

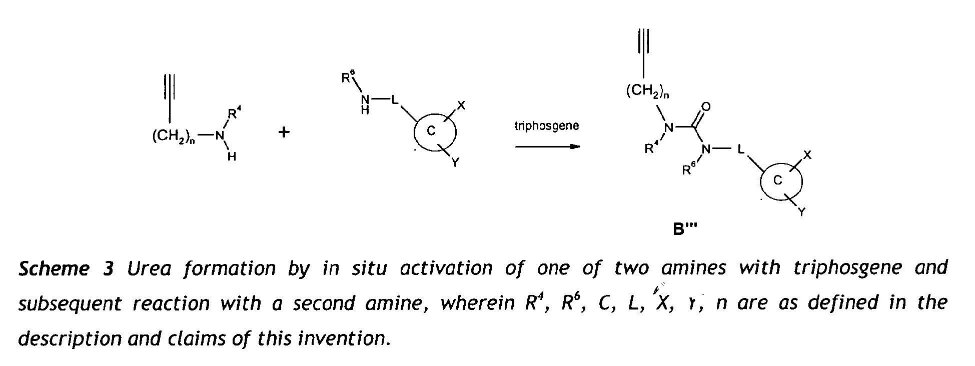

An alternative process of generating ureas of general formula **B'"** is depicted in **Scheme 3**. Urea formation starting from e.g. propargyl amine or homopropargyl amine may be achieved by coupling with a second functionalized amine via *in situ* transformation of one of the reacting amines into the respective carbamoyl chloride, aryl- or alkenylcarbamate (see for example J. Org. Chem. 2005, 70, 6960 and references cited therein). This process may provide an alternative to the formation and isolation of the respective isocyanate derived from one of the starting amines (see for example Tetrahedron Lett. 2004, 45, 4769). More particularly, ureas of formula B'" may be formed from amines and a suitable phosgene equivalents, preferably triphosgene, in an inert solvent, preferably acetonitrile, at temperatures ranging from -20 °C to room temperature, wherein room temperature is preferred (see also: J. Org. Chem. 1994, 59, 1937).

Processes for the preparation of functionalized (hetero)aryl amines are well known to the person skilled in the art. Starting from commercially available (hetero)aryl amines or nitro(hetero)arylenes well known transformations, including, but not limited to, e.g. alkylations, nucleophilic or electrophilic substitutions, acylations, halogenations, nitrations, sulfonylations, (transition) metal catalyzed couplings, metallations, rearrangements, reductions, and/or oxidations may be applied to prepare functionalized amines to be used in the urea formation step. In addition to specific procedures given in the following experimental section, detailed procedures may be found in the scientific and patent literature (see for example WO2005051366, WO2005110410, WO2005113494, and WO2006044823).

Processes for the preparation of functionalized (hetero)aryl sulfonylchlorides are as well known to the person skilled in the art. Introduction of sulfonyl groups may be accomplished by sulfonylation or by oxidation of thiols. Sulfonyl chlorides may be accessible in turn from sulfonic acids by reaction with e.g. thionyl chloride, sulfuryl chloride, PCl₅, POCl₃ or oxalyl chloride.

Processes for the preparation of functionalized (hetero)aryl carboxylic acids are well known to the person skilled in the art. Appropriate synthetic precursors include, but are not limited to, nitriles, carboxylic esters, benzylic alcohols, or benzylic halides. Benzylic nitriles and esters (and heteroaryl analogs thereof) can be efficiently alkylated at the benzylic position under basic conditions and subsequently hydrolyzed to the corresponding alkylated acids. Conditions for α-alkylations of nitriles and esters include, but are not limited to, the use of alkyl bromides or alkyl iodides as electrophiles under basic conditions in the presence or absence of a phase-transfer catalyst in a mono- or biphasic solvent system. Particularly, by using excess alkyl iodides as electrophilic species α,α-dialkylated nitriles are accessible. More particularly, by using 1,ω-dihaloalkyls as electrophiles cycloalkyl moieties can be installed at the benzylic position of nitriles and esters (J. Med. Chem. 1975, 18, 144; WO2003022852). The hydrolysis of nitriles to yield carboxylic acids can be accomplished, as known to the person skilled in the art, under acid or base-mediated conditions.

Incorporation of additional functionality into commercially available (hetero) aryl starting materials can be accomplished by a multitude of aromatic transformation reactions known to the person skilled in the art, including, but not limited to, e.g. electrophilic halogenations, electrophilic nitrations, Friedel-Crafts acylations, nucleophilic displacement of fluorine by oxygen nucleophiles and transformation of (hetero)aryl carboxylic acids into amides and subsequent reduction into benzylic amines, wherein the latter two methods are of particular relevance for the introduction of ether and/or aminomethylene side chains.

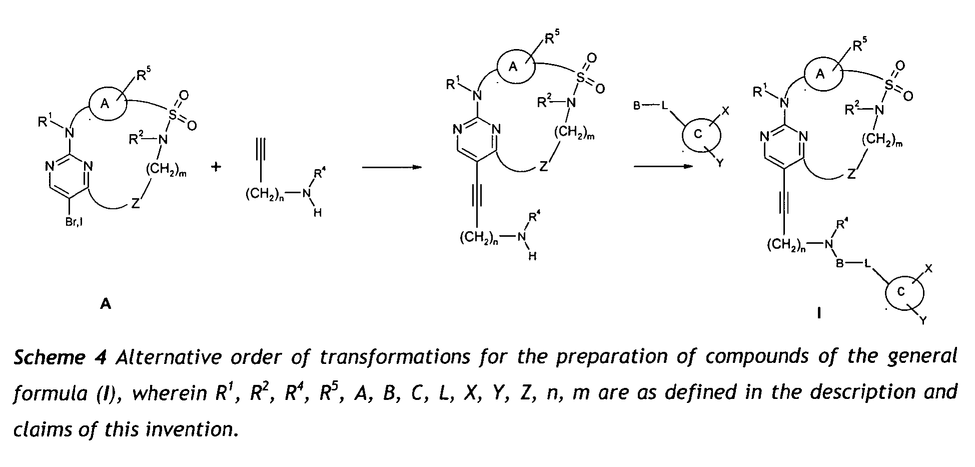

Alternatively to the process shown in **Scheme 1**, the order of transformations may be changed as exemplified in **Scheme 4**. Coupling of macrocyclic halides of general formula **A** with e.g. propargylic amine or homopropargylic amine under conditions as described above may be followed by appropriate derivatization of the lateral amino group with e.g. carboxylic acids, sulfonyl chlorides or isocyanates under conditions as described above. Introduction of R⁴ substituents may be achieved before or after any of the described transformations. In addition, protected analogues of propargyl amine or homopropargylamine may be used for the coupling to the macrocyclic halide, for example mono- or bis-BOC-protected propargyl amine or homopropargylamine or, more preferably, their respective phthalimides.

### Examples

In the subsequent paragraphs general procedures for the synthesis of the below mentioned specific example compounds are summarized.

### General Procedure 1 (GP1): Sonogashira coupling

PdCl₂(PPh₃)₂ (5 mol%), Cul (10 mol%), the respective macrocyclic halide (1.0 eq.) and alkyne (1.5 to 2 eq.) were weighed into a flame-dried Schlenk flask and subsequently set under an argon atmosphere. Dry DMF was added and the resulting solution was degassed for 5 minutes followed by addition of triethylamine (10 eq.). Stirring at room temperature was continued until TLC indicated complete turnover. The mixture was diluted with ethyl acetate, quenched with water. The watery layer was extracted twice with ethyl acetate, the combined organic layers were washed with aq. Na₂S₂O₃ solution and brine, dried and concentrated in vacuo. Pure Sonogashira products were obtained either by crystallization from acetone/hexane mixtures or by flash column chromatography optionally followed by preparative HPLC purification.

### General Procedure 2a (GP2a): Reaction of propargyl amine with isocyanates

A solution of propargyl amine in THF (0.3 M) was treated with the respective isocyanate (1.05 eq.) at room temperature under an atmosphere of nitrogen and stirred overnight. The mixture was evaporated and the resulting oil was triturated with hexane, filtered and the solid washed with additional ice-cold hexane to give the respective urea of sufficient purity for use without further purification.

### General Procedure 2b (GP2b): Reaction of homopropargyl amine hydrochloride with isocyanates

A solution of homopropargyl amine hydrochloride in THF (0.3 M) was treated under a nitrogen atmosphere with triethylamine (1.5 eq.) and subsequently with the respective isocyanate (1.05 eq.). The resulting mixture was stirred at room temperatur until TLC indicated complete turnover, evaporated and the resulting oil was triturated with hexane, filtered and the solid washed with additional ice-cold hexane to give the respective urea of sufficient purity for use without further purification.

### General Procedure 3a (GP 3a): Reaction of propargyl amine with sulfonyl chlorides

A solution of propargyl amine in acetonitrile (0.3 M) was treated with the respective sulfonyl chloride (1.05 eq.) and triethyl amine (1.5 eq.) at room temperature under an atmosphere of nitrogen and stirred until TLC indicated complete turnover. The mixture was quenched with water, extracted with ethyl acetate, dried and concentrated in vacuo. The resulting crude product was either triturated with hexane or re-crystallized from acetone/hexane to yield the desired sulfonamide, which was typically used without further purification.

### General Procedure 3b (GP 3b): Reaction of homopropargyl aminehydrochloride with sulfonyl chlorides

A solution of homopropargyl amine hydrochloride in acetonitrile (0.3 M) was treated under a nitrogen atmosphere with triethylamine (2.1 eq.) and subsequently portionwise with the respective sulfonyl chloride (1 eq.). The resulting mixture was stirred at room temperatur until TLC indicated complete turnover, quenched with water, extracted with ethyl acetate, dried and concentrated in vacuo. Trituration of the crude oil yielded the respective sulfonamide which was typically used without further purification.

### Preparation Example A:

### Production of 6-Bromo-13-thia-2,4,8,12,19-pentaaza-tricyclo[12.3.1.1^{3,7}]-nonadeca-1(18),3(19),4,6,14,16-hexaene 13,13-dioxide

### Step 1

### 3-Amino-N-[3-(5-bromo-2-chloro-pyrimidin-4-ylamino)-propyl]-benzenesulfonamide

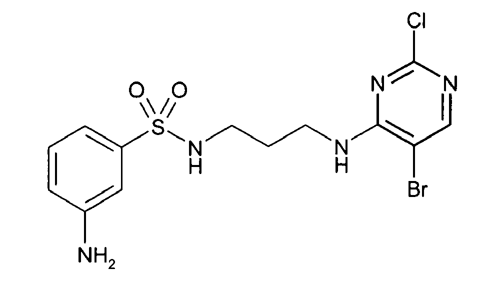

A solution of 1.35 g (2.99 mmol) of *N*-[3-(5-bromo-2-chloro-pyrimidin-4-ylamino)-propyl]-3-nitro-benzenesulfonamide in 100 ml of tetrahydrofuran was mixed under argon at room temperature with 15 ml of a 15% solution of Ti(III)Cl₃ in about 10% hydrochloric acid. After 17 hours, another 1 ml of the Ti(III)Cl₃ solution was added and the mixture was stirred for another 3 hours. 1 N NaOH solution was then added to the reaction mixture until pH > 10 and the slurry was suction filtered. The filter cake was washed 2x with 100 ml of ethyl acetate/MeOH (3:2). The filtrate was concentrated in vacuo and extracted with ethyl acetate (4x). The combined organic layers were washed with NaCl solution, dried (Na₂SO₄), filtered and concentrated by evaporation. The remaining residue was further purified by column chromatography (dichloromethane/ MeOH 95:5) to yield 624 mg (1.48 mmol, 49% yield) of the analytically pure product.

¹H-NMR (DMSO): 8.21 (s, 1H), 7.63 (t, 1H), 7.38 (t, 1H), 7.13 (t, 1H), 6.97 (m, 1H), 6.83 (m, 1H), 6.71 (m, 1H), 5.53 (s, 2H), 3.30 (m, 2H), 2.75 (m, 2H), 1.65 (m, 2H).

### Step 2

### 6-Bromo-13-thia-2,4,8,12,19-pentaaza-tricyclo[12.3.1.1^{3,7}]-nonadeca-1(18),3(19),4,6,14,16-hexaene 13,13-dioxide

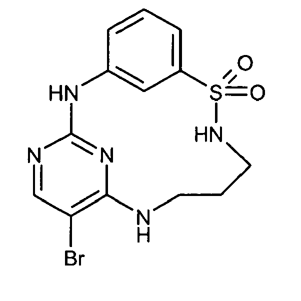

A solution of 200 mg (0.48 mmol) of 3-amino-N-[3-(5-bromo-2-chloro-pyrimidin-4-ylamino)-propyl]-benzenesulfonamide in acetonitrile/water/2-butanol (9.0 ml/1.0 ml/0.3 ml) was added via a syringe pump within 2.5 hours to a refluxing mixture of acetonitrile/water/4 N HCl in dioxane (45 ml/5 ml/0.6 ml). After completion of addition the resulting mixture is refluxed for another 3 hours. The reaction mixture was then allowed to reach room temperature and additional product precipitated upon standing at room temperature over night. The precipitate was filtered off, washed with water and dried in vacuum yielding 112 mg (0.31 mmol) of a white solid. The filtrate was concentrated in vacuo. The precipitate that is formed was again filtered, washed with water and dried to yield another 45 mg of the product. In total 157 mg of the product (0.41 mmol, 85% yield) were obtained in an analytically pure form.

¹H-NMR (DMSO): 10.45 (s, 1 H), 9.07 (s, 1 H), 8.35 (br, 1 H), 8.18 (s, 1 H), 7.78 (t, 1 H), 7.45 (m, 2H), 7.32 (m, 1H), 3.44 (m, 2H), 3.28 (m, 2H), 1.82 (m, 2H).
MS (ESI): [M+H]⁺ = 384 .

### Preparation Example B:

### Production of 6-Iodo-13-thia-2,4,8,12,19-pentaaza-thricyclo[12.3.1.1^{3,7}]nonadeca-1(18),3(19),4,6,14,16-hexaene 13,13-dioxide

### Step 1

### 3-Amino-N-[3-(2-chloro-5-iodo-pyrimidin-4-ylamino)-propyl]-benzene-sulfonamide

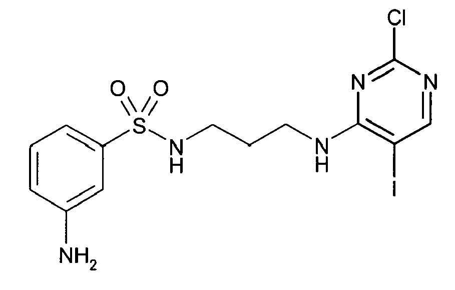

A solution of 9.95 g (20.0 mmol) of *N*-[3-(2-chloro-5-iodo-pyrimidin-4-ylamino)-propyl]-3-nitro-benzenesulfonamide in 660 mL of tetrahydrofuran was mixed under argon at room temperature with 100 mL of a 15% solution of Ti(III)Cl₃ in about 10% hydrochloric acid. After 2 hours, another 7 mL of the Ti(III)Cl₃ solution was added to the reaction mixture and stirring was continued for one hour. 1 N NaOH solution was then added to the reaction mixture until pH > 12 and the slurry was suction filtered over Celite. The filtrate was extracted with ethyl acetate (3x 400 mL), the combined organic layers were washed with brine (200 mL), and concentrated in vacuo. The filter cake was rewashed 4x with 500 ml of ethyl acetate/MeOH (3:2), followed by evaporation of the resulting washing fractions. The residues were combined and purified by column chromatography over silica (dichloromethane/ethyl acetate) to give 5.42 g (58 % yield) of the target compound.

¹H-NMR (DMSO, 300 MHz): 8.31 (s, 1 H), 7.39 (t, 1 H), 7.27 (t, 1 H), 7.16 (t, 1 H), 6.95 - 7.01 (m, 1H), 6.82 - 6.88 (m, 1H), 6.68 - 6.76 (m, 1H), 5.53 (s, 2H), 3.27 - 3.39 (m, 2H), 2.68 - 2.82 (m, 2H), 1.64 (mc, 2H).
ESI-MS: [M+H⁺] = 468 (³⁵Cl signal) + 470 (³⁷Cl signal)

### Step 2:

### 6-lodo-13-thia-2,4, 8,12,19-pentaaza-tricyclo[12.3.1.1^{3,7}]nonadeca-1(18),3(19),4,6,14,16-hexaene 13,13-dioxide

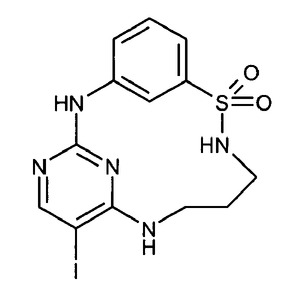

A solution of 2.34 g (5.00 mmol) of 3-amino-N-[3-(5-iodo-2-chloro-pyrimidin-4-ylamino)-propyl]-benzenesulfonamide in acetonitrile/water/2-butanol (94 mL/10.4 mL/3.1 mL) was added via a syringe pump within 3 hours to a refluxing mixture of acetonitrile/water/4 N HCl in dioxane (470 mL/52 mL/6.2 mL). After completion of addition the resulting mixture is refluxed for another 3 hours. The reaction mixture was then allowed to reach room temperature and additional product precipitated upon standing at room temperature over night. The precipitate was filtered off, washed with water and dried in vacuum yielding 1.71 g (79% yield) of a white solid.

¹H-NMR (DMSO, 300 MHz): 10.81 (s, 1H), 9.02 (s, 1H), 8.30 - 8.38 (m, 1H), 8.27 (s, 1 H), 7.82 (t, 1 H), 7.43 - 7.56 (m, 2H), 7.29 - 7.40 (m, 1 H), 3.38 - 3.52 (m, 2H), 3.21 - 3.36 (m, 2H), 1.72 - 1.90 (m, 2H).
ESI-MS: [M+H⁺] = 432.

The following Intermediate A to N were prepared by reaction of propargyl amine or homopropargyl amine hydrochloride with the respective isocyanate or sulfonyl chloride in analogy to the general procedures GP 2a, 2b, 3a or 3b as stated below the respective structures.

| **Intermediate** | **Structure** | **Name** | **Analytical data** |
|---|---|---|---|
| **A** | 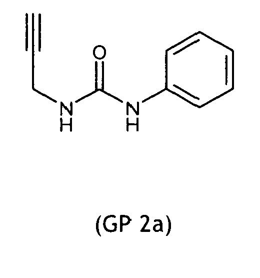 | 1-(Prop-2-ynyl)-3-(phenyl)urea | ¹H-NMR (DMSO, 300 MHz): 8.53 (s, 1 H); 7.34-7.36 (m, 2 H); 7.19 (t, 2 H); 6.87 (t, 1 H); 6.40 (t, 1 H); 3.89 (dd, 2 H); 3.07 (t, 1 H). MS (ESI): [M+H]⁺ = 175. |
| **B** | 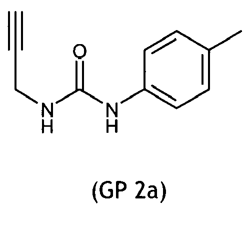 | 1-(Prop-2-ynyl)-3-(4-methylphenyl)urea | ¹H-NMR (DMSO, 400 MHz): 8.41 (s, 1 H); 7.23 (d, 2 H); 6.99 (d, 2 H); 6.34 (t, 1 H); 3.83 (dd, 2 H); 3.06 (t, 1 H); 2.17 (s, 3 H). |
| **C** | 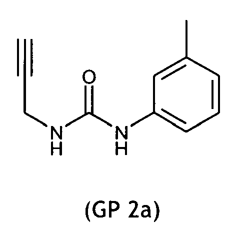 | 1-(Prop-2-ynyl)-3-(3-methylphenyl)urea | ¹H-NMR (DMSO, 400 MHz): 8.44 (s, 1 H); 7.13 - 7.18 (m, 2 H); 7.06 (t, 1 H); 6.69 (d, 1 H); 6.38 (t, 1 H); 3.87 (dd, 2 H); 3.06 (t, 1 H); 2.20 (s, 3 H). |
| **D** | 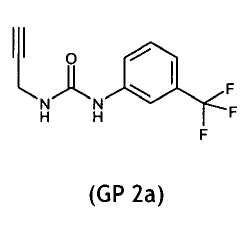 | 1-Prop-2-ynyl-3-(3-trifluoromethyl-phenyl)-urea | ¹H-NMR (DMSO, 300 MHz): 8.97 (s, 1 H); 7.92 (s, 1 H); 7.48 (d, 1 H); 7.41 (t, 1 H); 7.21 (d, 1 H); 6.60 (t, 1 H); 3.85 (dd, 2 H); 3.08 (t, 1 H). |
| **E** | 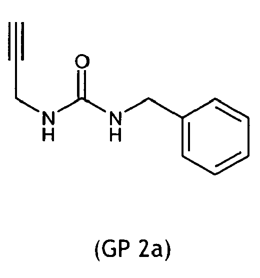 | 1-Benzyl-3-prop-2-ynyl-urea | ¹H-NMR (DMSO, 300 MHz): 7.15 - 7.30 (m, 5 H); 6.43 (t, 1 H); 6.23 (t, 1H); 4.17 (d, 2 H); 3.78 (dd, 2 H); 3.01 (t, 1 H).MS (ESI): [M+H]⁺ = 189. |
| **F** | 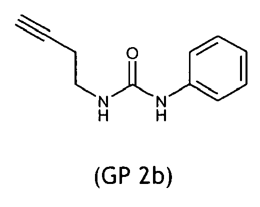 | 1-(But-3-ynyl)-3-(phenyl)urea | ¹H-NMR (DMSO, 300 MHz): 8.54 (s, 1 H); 7.35 (d, 2 H); 7.17 (t, 2 H); 6.85 (t, 1 H); 6.23 (t, 1 H); 3.18 (m, 2 H); 2.83 (t, 1 H); 2.29 (td, 2 H). |
| **G** | 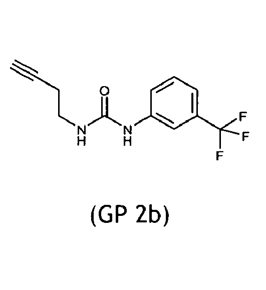 | 1-But-3-ynyl-3-(3-trifluoromethyl-phenyl)-urea | ¹H-NMR (DMSO, 300 MHz): 9.25 (s, 1 H); 7.98 (br. s, 1 H); 7.52 (d, 1 H); 7.45 (t, 1 H); 7.23 (d, 1 H); 6.56 (t, 1 H); 3.20 - 3.26 (m, 2 H); 2.88 (t, 1 H); 2.34 (td, 2 H). MS (ESI): [M+H]⁺ = 257. |
| **H** | 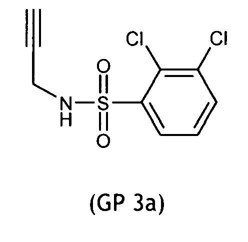 | 2, 3-Dichtoro-*N*-prop-2-ynyl-benzene-sulfonamide | ¹H-NMR (DMSO, 300 MHz): 8.50 (t, 1 H); 7.88 - 7.95 (m, 2 H); 7.52 (t, 1 H); 3.76 (dd, 2 H); 2.95 (t, 1 H). |
| **I** | 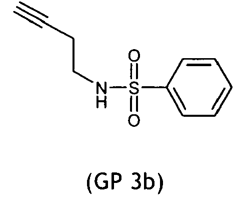 | *N*-But-3-ynyl-benzene-sulfonamide | ¹H-NMR (DMSO, 400 MHz): 7.81 (t, 1 H); 7.75 - 7.78 (m, 2 H); 7.54 - 7.63 (m, 3 H); 2.79 - 2.84 (m, 3 H); 2.23 (dt, 2 H). |
| **J** | 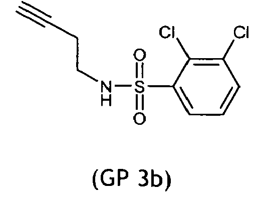 | *N*-But-3-ynyl-2,3-dichloro-benzene-sulfonamide | ¹H-NMR (DMSO, 300 MHz): 8.24 (br. s, 1 H); 7.87-7.94 (m, 2 H); 7.52 (t, 1 H); 2.97 (t, 2 H); 2.73 (t, 1 H); 2.25 (td, 2 H). |
| **K** | 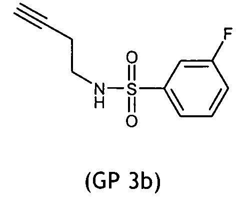 | N-But-3-ynyl-3-fluoro-benzene-sulfonamide | ¹H-NMR (DMSO, 300 MHz): 7.95 (br. s, 1 H); 7.45-7.67 (m, 4 H); 2.85 (t, 2 H); 2.80 (t, 1 H); 2.24 (dt, 2 H). |
| **L** | 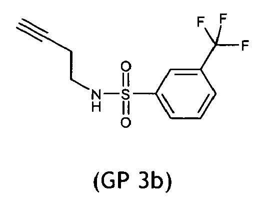 | *N*-But-3-ynyl-3-trifluoromethyl-benzene-sulfonamide | ¹H-NMR (DMSO, 400 MHz): 8.00 - 8.09 (m, 4 H); 7.83 (t, 1 H); 2.87 (t, 2 H); 2.78 (t, 1 H); 2.24 (dt, 2 H). |
| **M** | 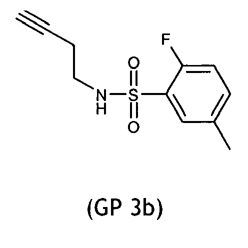 | N-But-3-ynyl-2-fluoro-5-methyl-benzene-sulfonamide | ¹H-NMR (DMSO, 400 MHz): 8.01 (t, 1 H); 7.56 (dd, 1 H); 7.43 - 7.47 (m, 1 H); 7.28 (dd, 1 H); 2.94 (q, 2 H); 2.78 (t, 1 H); 2.25 (dt, 2 H). |
| **N** | 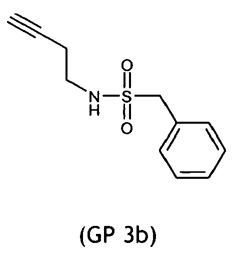 | *N*-But-3-ynyl-C-phenyl-methane-sulfonamide | ¹H-NMR (DMSO, 400 MHz): 7.23 - 7.36 (m, 6 H); 4.31 (s, 2 H); 2.92 - 2.97 (m, 2 H); 2.84 (t, 1 H); 2.25 (dt, 2 H). |

### Intermediate O

### Preparation of 1-Phenyl-cyclopropanecarboxylic acid prop-2-ynylamide

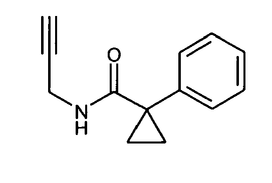

1.5 g Phenylcyclopropylcarboxylic acid chloride (8.3 mmol, 1.2 eq.; prepared from the corresponding carboxylic acid by treatment with thionyl chloride followed by concentration in vacuo) were dissolved in 30 mL pyridine and treated at rt with 381 mg propargyl amine (6.92 mmol, 1.0 eq.). This mixture was stirred at rt for 2d, concentrated in vacuo and the residue was purified by flash column chromatography to yield 950 mg (57%) of a white solid.

¹H-NMR (DMSO, 400 MHz): 7.23 - 7.35 (m, 5 H); 7.08 (t, 1 H); 3.71 (dd, 2 H); 2.95 (t, 1 H); 1.30 (m, 2 H); 0.94 (m, 2 H).
MS (ESI): [M+H]⁺ = 200.

### Intermediate P

### Preparation of 1-Phenyl-cyclopropanecarboxylic acid but-3-ynylamide

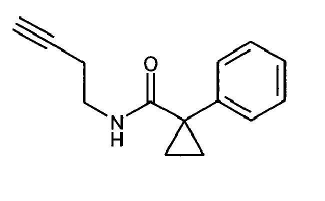

422 mg Homopropargylamine hydrochloride (4 mmol, 1 eq.) were suspended in 12 mL acetonitrile and treated with 1.11 mL triethylamine (8 mmol, 2 eq.) and subsequently portionwise with 722 mg phenylcyclopropylcarboxylic acid chloride (see above, 4 mmol, 1 eq.). This mixture was stirred at rt for 16h, diluted with ethyl acetate and quenched with water. The aqueous layer was extracted with ethyl acetate twice, the combined organic layers were washed with brine, dried and concentrated in vacuo to yield the crude amide as an off-white solid in quantitative yield.

¹H-NMR (DMSO, 400 MHz): 7.22 - 7.33 (m, 5 H); 6.76 (t, 1 H); 3.08 (q, 2 H); 2.73 (t, 1 H); 2.18 (dt, 2 H); 1.28 - 1.30 (m, 2 H); 0.91 - 0.93 (m, 2 H).
MS (ESI): [M+H]⁺ = 214.

The following example compounds were prepared by Sonogashira couplings according to the general procedure GP 1 from 6-lodo-13-thia-2,4,8,12,19-pentaaza-tricyclo[12.3.1.1^{3,7}]nonadeca-1(18),3(19),4,6,14,16-hexaene 13,13-dioxide and the respective alkynes.

| **Example** | **Structure (Procedure)** | | **Name** | **Analytical date** |
|---|---|---|---|---|
| **1** | 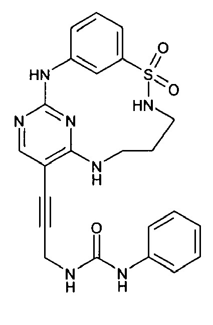 | | 1-[3-(13,13-Dioxo-13λ⁶-thia-2,4,8,12,19-pentaaza-tricyclo[12.3.1.1^{3,7}]-nonadeca-1(18),3(19),4,6,14,16 -hexaen-6-yl)-prop-2-ynyl]-3-phenyl-urea | ¹H-NMR (DMSO, 300 MHz): 10.16 (s, 1 H); 9.17 (s, 1 H); 8.68 (s, 1 H); 8.02 (br. s, 1 H); 7.92 (br. s, 1 H); 7.75 (t, 1 H); 7.17 - 7.44 (m, 7 H); 6.88 (t, 1 H); 6.55 (t, 1 H); 4.14 (d, 2 H); 3.34 - 3.46 (m, 2 H); 3.20 - 3.29 (m, 2 H); 1.72- 1.81 (m, 2 H). MS (ESI): [M+H]⁺ = 478. |
| **2** | 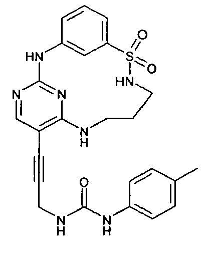 | | 1-[3-(13,13-Dioxo-13λ⁶ -thia-2,4,8,12,19-pentaaza-tricyclo[12.3.1.1^{3,7}]-nonadeca-1 (18),3(19),4,6,14,16 -hexaen-6-yl)-prop-2-ynyl]-3-p-tolyl-urea | ¹H-NMR (DMSO, 300 MHz): 9.71 (s, 1 H); 9.34 (s, 1 H); 8.49 (s, 1 H); 7.95 (s, 1 H); 7.71 (t, 1 H); 7.23 - 7.39 (m, 6 H); 7.00 (d, 2 H); 6.45 (t, 1H) ; 4.12 (d,2 H); 3.34 - 3.44 (m, 2 H); 3.21-3.28 (m, 2 H); 2.19 (s, 3 H); 1.71 - 1.81 (m, 2 H). MS (ESI): [M+H]⁺ = 492. |
| **3** | 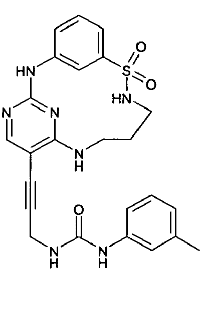 | | 1-[3-(13,13-Dioxo-13λ⁶-thia-2,4,8,12,19-pentaaza-tricyclo[12.3.1.1^{3,7}]-nonadeca-1 (18),3(19),4,6,14,16 -hexaen-6-yl)-prop-2-ynyl]-3-m-tolyl-urea | ¹H-NMR (DMSO, 300 MHz): 9.71 (s, 1 H); 9.34 (s, 1 H); 8.54 (s, 1 H); 7.95 (s, 1 H); 7.71 (t, 1 H); 7.33-7.40 (m, 2 H); 7.14-7.28 (m, 4 H); 7.07 (t, 1 H); 6.71 (d, 1 H); 6.49 (t, 1 H); 4.12 (d, 2 H); 3.34 - 3.44 (m, 2 H); 3.22 - 3.28 (m, 2 H); 2.21 (s, 3 H); 1.72 - 1.83 (m, 2 H). MS (ESI): [M+H]⁺ = 492. |
| **4** | 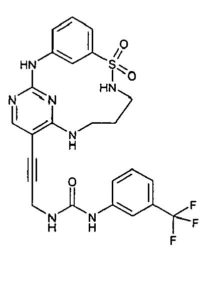 | | 1-[3-(13,13-Dioxo-13λ⁶-thia-2,4,8,12,19-pentaaza-tricyclo[12.3.1.1^{3,7}]-nonadeca-1 (18),3(19),4,6,14,16 -hexaen-6-yl)-prop-2-ynyl]-3-(3-trifluoromethyl-phenyl)-urea | ¹H-NMR (DMSO, 300 MHz): 9.72 (s, 1 H); 9.34 (s, 1 H); 9.02 (s, 1 H); 7.94 - 7.97 (m, 2 H); 7.72 (t, 1 H); 7.50 (br. d, 1 H); 7.43 (br. t, 1 H); 7.21 - 7.38 (m, 5H); 6.67 (t, 1 H); 4.15 (d, 2 H); 3.34 - 3.44 (m, 2 H); 3.21 - 3.27 (m, 2 H); 1.73 - 1.83 (m, 2 H). MS (ESI): [M+H]⁺ = 546. |
| **5** | 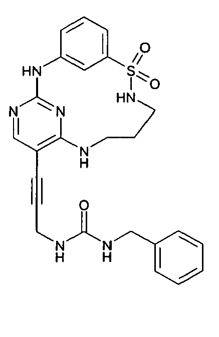 | | 1-Benzyl-3-[3-(13,13-dioxo-13λ⁶-thia-2,4,8,12,19-pentaaza-tricyclo)[12.3.1.1^{3,7}]-nonadeca-1(18),3(19),4,6,14,16 -hexaen-6-yl)-prop-2-ynyl]-urea | ¹H-NMR (DMSO, 400 MHz): 10.21 (s, 1 H); 9.17 (s, 1 H); 7.93 - 8.01 (m, 2 H); 7.75 (t, 1 H); 7.36-7.43 (m, 2 H); 7.16-7.31 (m, 6 H); 6.61 (t, 1 H); 6.37 (br. s, 1 H); 4.30 (d, 2 H); 4.07 (s, 2 H); 3.34 - 3.41 (m, 2 H); 3.22 - 3.29 (m, 2 H); 1.73-1.80 (m, 2 H). MS (ESI): [M+H]⁺ = 492. |
| **6** | 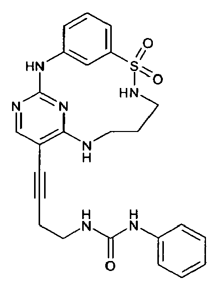 | | 1-[4-(13,13-Dioxo-13λ⁶-thia-2,4,8,12,19-pentaaza-tricyclo[12.3.1.1^{3,7}]-nonadeca-1(18),3(19),4,6,14,16 -hexaen-6-yl)-but-3-ynyl]-3-phenyl-urea | ¹H-NMR (DMSO, 400 MHz): 10.12 (s, 1 H); 9.15 (s, 1 H); 8.64 (s, 1 H); 7.98 (s, 1 H); 7.74 (t, 2 H); 7.40 (t, 1 H); 7.33 - 7.36 (m, 3 H); 7.27 (d, 1 H); 7.20 (t, 2 H); 6.88 (t, 1 H); 6.42 (t, 1 H); 3.30 - 3.37 (m, 4 H); 3.13 - 3.17 (m, 2 H); 2.58 (t, 2 H); 1.57 - 1.64 (m, 2 H). MS (ESI): [M+H]⁺ = 492. |
| **7** | 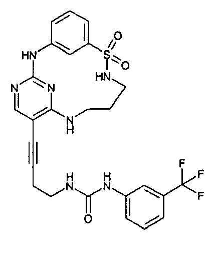 | | 1-[4-(13,13-Dioxo-13λ⁶-thia-2,4,8,12,19-pentaaza-tricyclo[12.3.1.1^{3,7}]-nonadeca-1(18),3(19),4,6,14,16 -hexaen-6-yl)-but-3-ynyl]-3-(3-trifluoromethyl-phenyl)-urea | ¹H-NMR (DMSO, 300 MHz): 9.68 (s, 1 H); 9.32 (s, 1 H); 8.99 (s, 1 H); 7.89 - 7.94 (m, 2 H); 7.71 (t, 1 H); 7.51 (d, 1 H); 7.43 (t, 1H); 7.33 (d, 1 H); 7.19 - 7.27 (m, 3H); 7.14 (t, 1 H); 6.51 (t, 1 H); 3.30 - 3.38 (m, 4 H); 3.12-3.19(m, 2H);2.59(t, 2 H); 1.60-1.71(m,2H). MS (ESI): [M+H]⁺ = 560 |
| **8** | 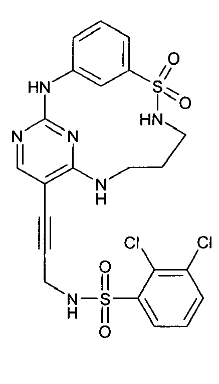 | | 2,3-Dichloro-N-[3-(13,13-dioxo-13λ⁶-thia-2,4,8,12,19-pentaaza-tricyclo[12.3.1.1^{3,7}]-nonadeca-1(18),3(19),4,6,14,16 -hexaen-6-yl)-prop-2-ynyl]-benzene-sulfonamide | ¹H-NMR (DMSO, 400 MHz): 9.77 (s, 1 H); 9.32 (s, 1 H); 8.44 (t, 1 H); 7.99 (d, 1 H); 7.79 (d, 1 H); 7.73 (t, 1 H); 7.53 (s, 1 H); 7.49 (t, 1 H); 7.36 (t, 1 H); 7.23 - 7.29 (m, 2 H); 7.05 (t, 1 H); 4.03 (d, 2 H); 3.32 - 3.40 (m, 2 H); 3.24 - 3.30 (m, 2 H); 1.74 - 1.82 (m, 2 H). MS (ESI): [M+H]⁺ = 567. |
| **9** | 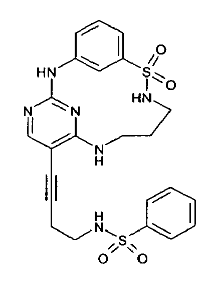 | | N-[4-(13,13-Dioxo-13λ⁶-thia-2,4,8,12,19-pentaaza-tricyclo[12.3.1.1^{3,7}]-nonadeca-1(18),3(19),4,6,14,16 -hexaen-6-yl)-but-3-ynyl]-benzene-sulfonamide | ¹H-NMR (DMSO, 400 MHz): 9.96 (br. s, 1 H); 9.21 (br. s, 1 H); 7.92 (s, 1 H); 7.91 (t, 1 H); 7.72 - 7.80 (m, 3 H);7.53 - 7.65 (m, 4 H); 7.39 (t, 1 H); 7.33 (d, 1 H); 7.27 (d, 1 H); 3.35 - 3.43 (m, 2 H); 3.23 - 3.29 (m, 2 H); 2.96 (q, 2 H); 2.53 (t, 2 H); 1.73 -1.83(m,2H). MS (ESI): [M+H]⁺ = 513. |
| **10** | 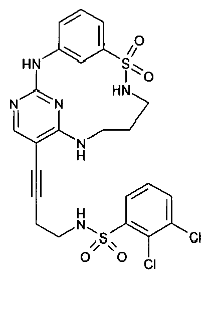 | | 2,3-Dichloro-N-[4-(13,13-dioxo-13λ⁶-thia-2,4,8,12,19-pentaaza-tricyclo[12.3.1.1^{3,7}]-nonadeca-1(18),3(19),4,6,14,16 -hexaen-6-yl)-but-3-ynyl]-benzene-sulfonamide | ¹H-NMR (DMSO, 400 MHz): 9.71 (s, 1 H); 9.36 (s, 1 H); 8.33 (br. s, 1 H); 7.95 (dd, 1 H); 7.84 - 7.88 (m, 2 H); 7.74 (t, 1 H); 7.51 (t, 1 H); 7.35 (t, 1 H); 7.24 - 7.28 (m, 2 H); 7.15 (m, 1 H); 3.35 - 3.43 (m, 2 H); 3.23 - 3.29 (m, 2 H); 3.09-3.15 (m, 2 H); 2.53 (t, 2 H); 1.76 - 1.83 (m, 2 H). MS (ESI): [M]⁺ = 581 and [M+2]⁺= 583. |
| **11** | 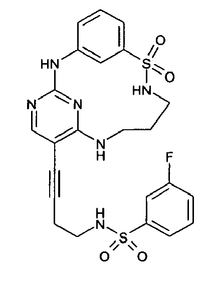 | | N-[4-(13,13-Dioxo-13λ⁶-thia-2,4,8,12,19-pentaaza-tricyclo[12.3.1.1^{3,7}]-nonadeca-1 (18),3(19),4,6,14,16 -hexaen-6-yl)-but-3-ynyl]-3-fluoro-benzene-sulfonamide | ¹H-NMR (DMSO, 400 MHz): 10.04 (br. s, 1 H); 9.20 (m, 1 H); 8.06 (t, 1 H); 7.94 (s, 1 H); 7.75 (t, 1 H); 7.56 - 7-66 (m, 4 H); 7.45 - 7.51 (m, 1 H); 7.40 (t, 1 H); 7.35 (d, 1 H); 7.28 (d, 1 H); 3.36 - 3.42 (m, 2 H); 3.24 - 3.28 (m, 2 H); 3.02 (dt, 2 H); 2.55 (t, 2 H); 1.76- 1.83 (m, 2 H). MS (ESI): [M+H]⁺ = 531. |
| **12** | 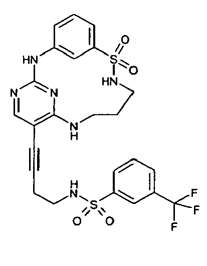 | | N-[4-(13,13-Dioxo-13λ⁶-thia-2,4,8,12,19-pentaaza-tricyclo[12.3.1.1^{3,7}]-nonadeca-1 (18),3(19),4,6,14,16 -hexaen-6-yl)-but-3-ynyl]-3-trifluoromethyl-benzene-sulfonamide | ¹H-NMR (DMSO, 400 MHz): 10.12 (br. s, 1 H); 9.18 (br. s, 1 H); 8.18 (t, 1 H); 8.10 (dd, 1 H); 8.07 (s, 1 H); 8.01 (d, 1 H); 7.94 (s, 1 H); 7.82 (t, 1 H); 7.75 (t, 1 H); 7.69 - 7.75 (m, 1 H); 7.41 (t, 1 H); 7.34-7.37 (m, 1 H); 7.28 (d, 1 H); 3.36 - 3.42 (m, 2 H); 3.24- 3.29 (m, 2 H); 3.03 (q, 2 H); 2.55 (t, 2 H); 1.75-1.83 (m, 2 H). MS (ESI): [M+H]⁺ = 581. |
| **13** | 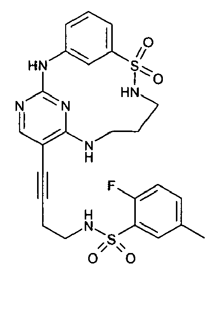 | | N-[4-(13,13-Dioxo-13λ⁶-thia-2,4,8,12,19-pentaaza-tricyclo[12.3.1.1^{3,7}]-nonadeca-1(18),3(19),4,6,14,16 -hexaen-6-yl)-but-3-ynyl]-2-fluoro-5-methyl-benzene-sulfonamide | ¹H-NMR (DMSO, 400 MHz): 10.19 (br. s, 1 H); 9.16 (br. s, 1 H); 8.13 (t, 1H); 7.95 (s, 1H) ; 7.80 (br. s, 1 H); 7.75 (t, 1 H); 7.57 (dd, 1 H); 7.36 - 7.46 (m, 3 H); 7.24 - 7.30 (m, 2 H); 3.36 - 3.42 (m, 2 H); 3.24 - 3.28 (m, 2 H); 3.09 (q, 1 H); 2.56 (t, 2 H); 2.30 (s, 3 H); 1.75 - 1.83 (m, 2 H). MS (ESI): [M+H]⁺ = 545. |
| **14** | 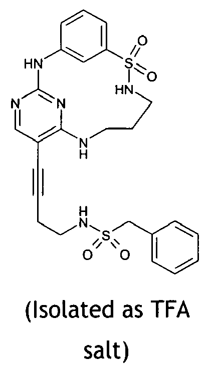 | | N-[4-(13,13-Dioxo-13λ⁶-thia-2,4,8,12,19-pentaaza-tricyclo[12.3.1.1^{3,7}]-nonadeca-1(18),3(19),4,6,14,16 -hexaen-6-yl)-but-3-ynyl]-C-phenyl-methane-sulfonamide | ¹H-NMR (DMSO, 400 MHz): 10.09 (br. s, 1 H); 9.18 - 9.20 (m, 1 H); 7.99 (s, 1 H); 7.72 - 7.74 (m, 2 H); 7.27 - 7.43 (m, 9 H); 4.35 (s, 2 H); 3.36-3.43 (m, 2 H); 3.24-3.28 (m, 2 H); 3.11 (q, 2 H); 2.57 (t, 2 H); 1.75 - 1.83 (m, 2 H). MS (ESI): [M+H]⁺ = 527. |
| **15** | 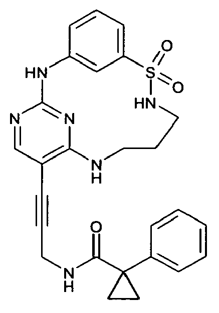 | | 1-Phenyl-cyclopropanecarboxy lic acid [3-(13,13-dioxo-13λ⁶-thia-2,4,8,12,19-pentaaza-tricyclo[12.3.1.1^{3,7}]-nonadeca-1 (18),3(19),4,6,14,16 -hexaen-6-yl)-prop-2-ynyl]-amide | ¹H-NMR (DMSO, 300 MHz): 9.74 (s, 1 H); 9.34 (s, 1 H); 7.91 (s, 1 H); 7.75 (t, 1H); 7.23 - 7.38 (m, 9 H); 7.16 (t, 1 H); 4.04 (d, 2 H); 3.32-3.41 (m, 2 H); 3.23-3.28 (m, 2 H); 1.72 - 1.84 (m, 2 H); 1.34 (m, 2 H); 0.97 (m, 2 H). MS (ESI): [M+H]⁺ = 503. |
| **16** | 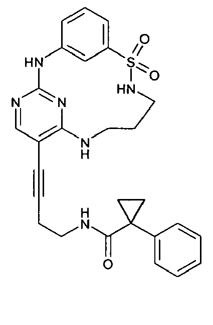 | | 1-Phenyl-cyclopropanecarboxy lic acid [4-(13,13-dioxo-13λ⁶-thia-2,4,8,12,19-pentaaza-tricyclo[12.3.1.1^{3,7}]n onadeca-1 (18),3(19),4,6,14,16 -hexaen-6-yl)-but-3-ynyl]-amide | ¹H-NMR (DMSO, 300 MHz): 10.03 (br. s, 1 H); 9.28 (br. s, 1 H); 7.96 (s, 1 H); 7.80 (t, 1 H); 7.23 - 7.66 (m, 9 H); 6.99 (t, 1 H); 3.38 - 3.48 (m, 2 H); 3.26 - 3.34 (m, 4 H); 2.47 - 2.60 (signal overlap by solvent); 1.79 - 1.90 (m, 2 H); 1.34 - 1.38 (m, 2 H); 0.97 - 1.02(m,2H). MS (ESI): [M+H]⁺ = 517. |

The following Example Compounds can be obtained using the methods described before e.g. by Sonogashira coupling of 6-lodo-13-thia-2,4,8,12,19-pentaaza-tricyclo[12.3.1.1^{3,7}]nonadeca-1(18),3(19),4,6,14,16-hexaene 13,13-dioxide with the respective alkynes, which are accessible as desribed above.

| | | | |
|---|---|---|---|
| 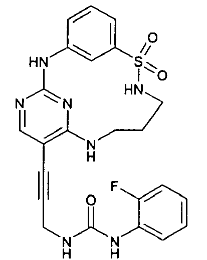 | 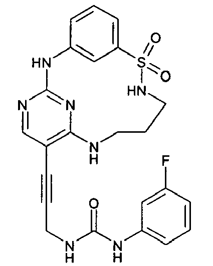 | 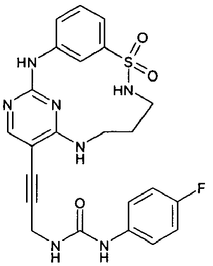 | 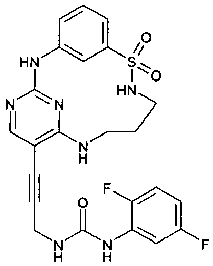 |
| **Example 17** | **Example 18** | **Example 19** | **Example 20** |
| 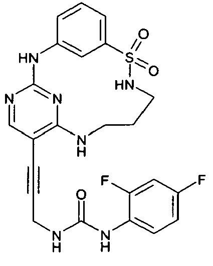 | 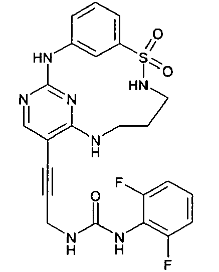 | 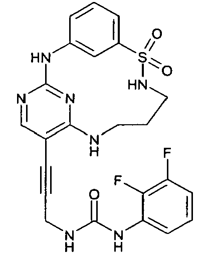 | 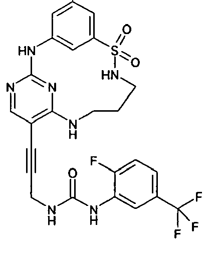 |
| **Example 21** | **Example 22** | **Example 23** | **Example 24** |
| 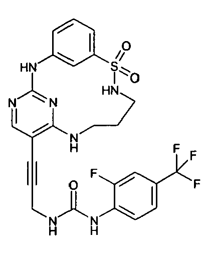 | 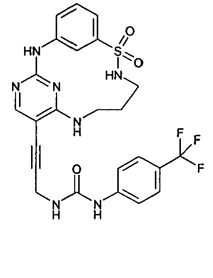 | 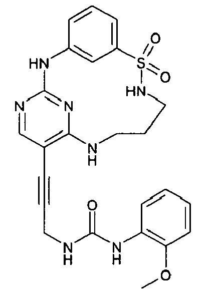 | 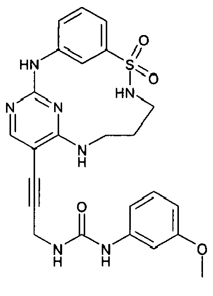 |
| **Example 25** | **Example 26** | **Example 27** | **Example 28** |
| 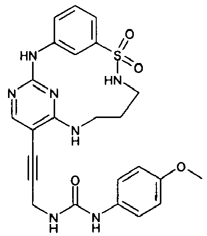 | 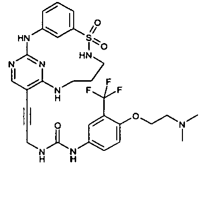 | 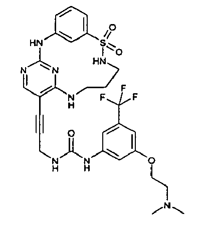 | 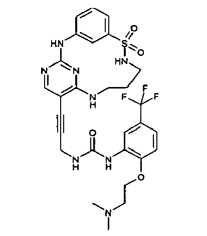 |
| **Example 29** | **Example 30** | **Example 31** | **Example 32** |
| 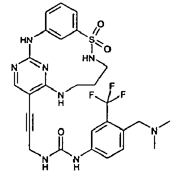 | 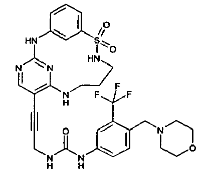 | 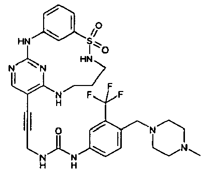 | 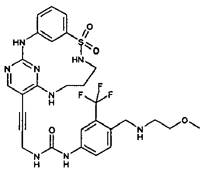 |
| **Example 33** | **Example 34** | **Example 35** | **Example 36** |
| 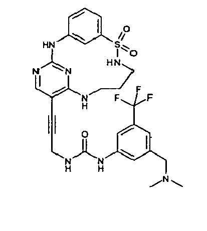 | 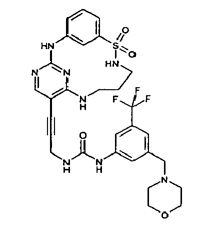 | 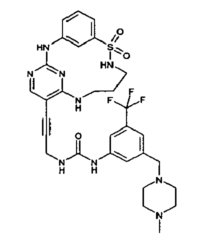 | 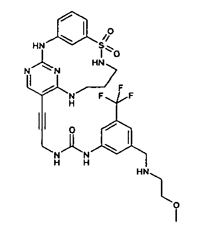 |
| **Example 37** | **Example 38** | **Example 39** | **Example 40** |
| 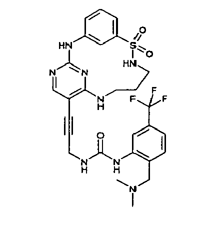 | 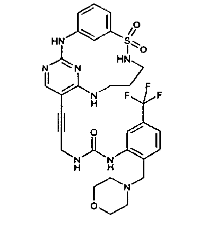 | 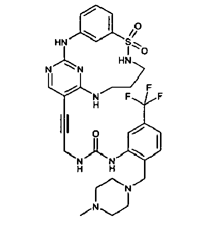 | 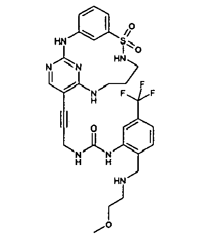 |
| **Example 41** | **Example 42** | **Example 43** | **Example 44** |
| 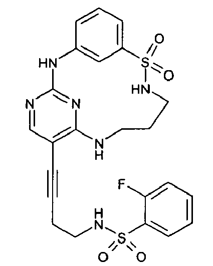 | 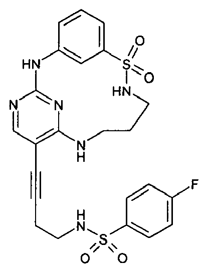 | 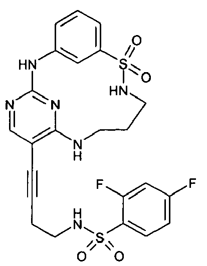 | 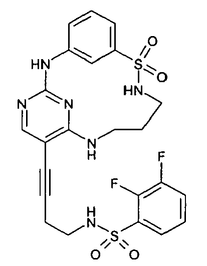 |
| **Example 45** | **Example 46** | **Example 47** | **Example 48** |
| 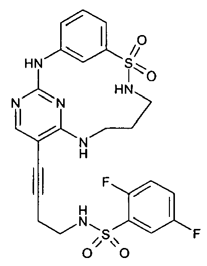 | 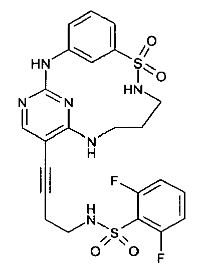 | 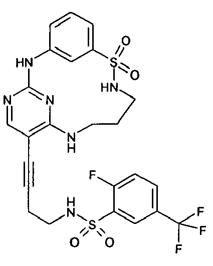 | 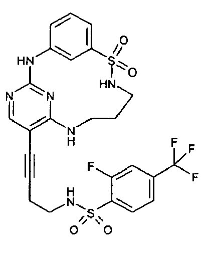 |
| **Example 49** | **Example 50** | **Example 51** | **Example 52** |
| 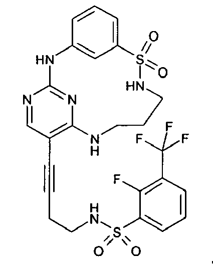 | 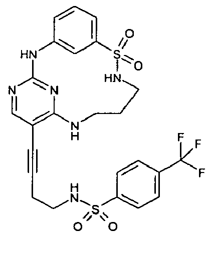 | 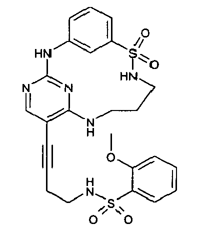 | 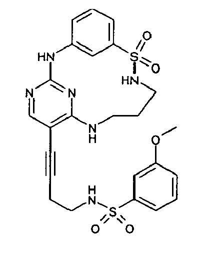 |
| **Example 53** | **Example 54** | **Example 55** | **Example 56** |
| 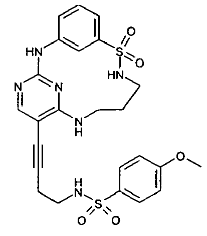 | 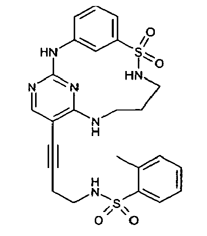 | 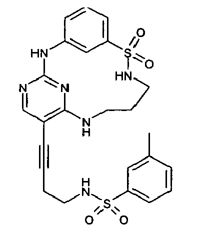 | 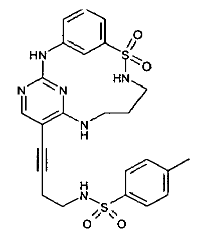 |
| **Example 57** | **Example 58** | **Example 59** | **Example 60** |
| 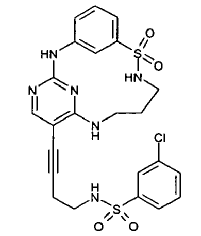 | 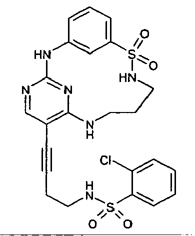 | 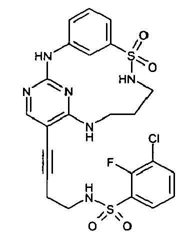 | 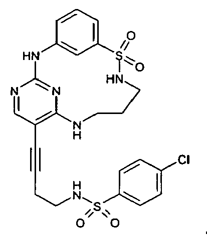 |
| **Example 61** | **Example 62** | **Example 63** | **Example 64** |
| 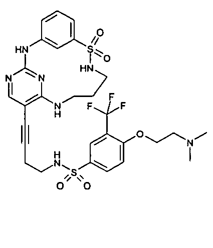 | 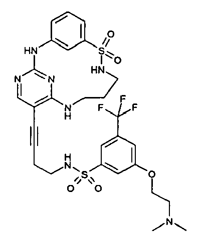 | 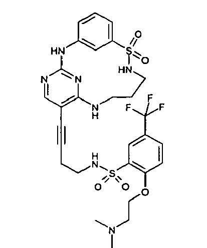 | 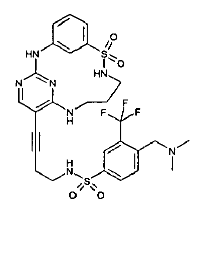 |
| **Example 65** | **Example 66** | **Example 67** | **Example 68** |
| 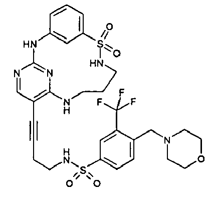 | 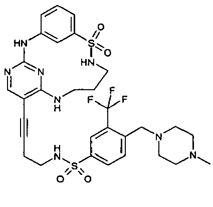 | 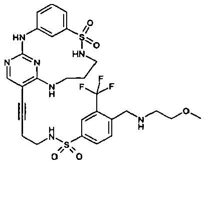 | 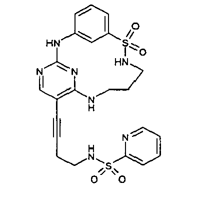 |
| **Example 69** | **Example 70** | **Example 71** | **Example 72** |
| 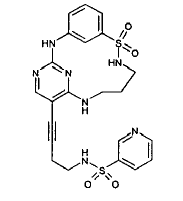 | 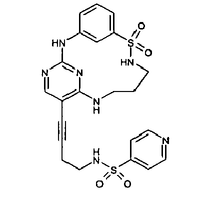 | 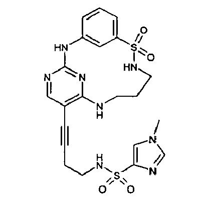 | 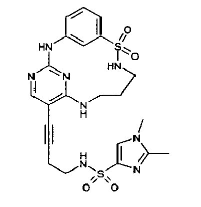 |
| **Example 73** | **Example 74** | **Example 75** | **Example 76** |
| 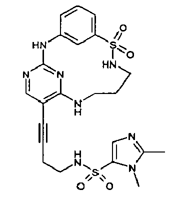 | 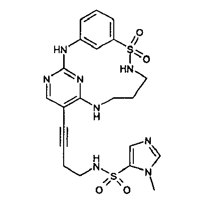 | 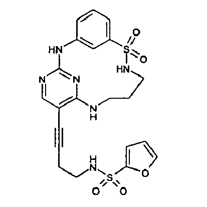 | 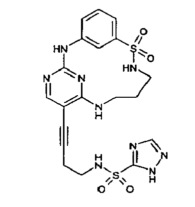 |
| **Example 77** | **Example 78** | **Example 79** | **Example 80** |
| 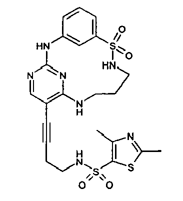 | 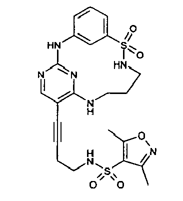 | 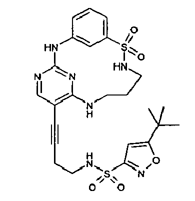 | 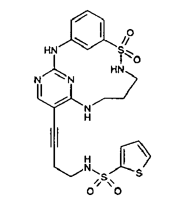 |
| **Example 81** | **Example 82** | **Example 83** | **Example 84** |
| 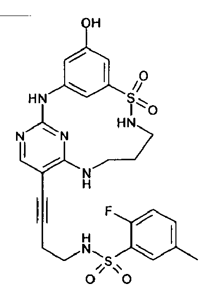 | 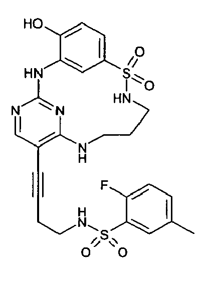 | 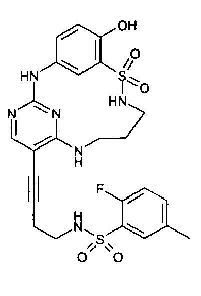 | 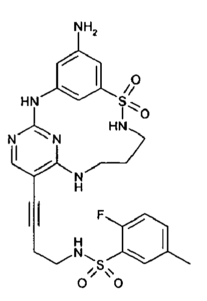 |
| **Example 85** | **Example 86** | **Example 87** | **Example 88** |
| 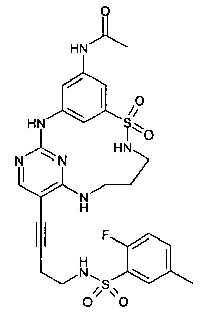 | 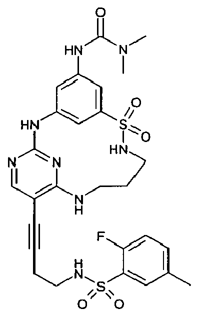 | 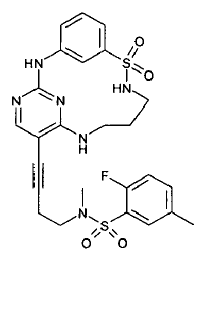 | 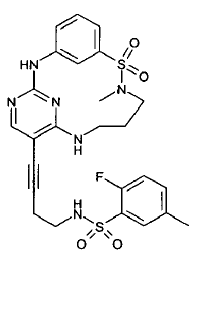 |
| **Example 89** | **Example 90** | **Example 91** | **Example 92** |

### BIOLOGICAL DATA

### Assay 1: Tie2 ELISA Assay

Cellular activity of compounds of the present invention as inhibitors of Tie2 kinase activity was measured employing a Tie2 ELISA assay as described in the following paragraphs. Herein CHO cell-cultures, which are stably transfected by known techniques with Tie2 using DHFR deficiency as selection marker, are stimulated by angiopoietin-2. The specific autophosphorylation of Tie2 receptors is quantified with a sandwich-ELISA using anti-Tie2 antibodies for catch and anti-phosphotyrosine antibodies coupled to HRP for detection.

### Materials:

96well tissue culture plate, sterile, Greiner
96well FluoroNunc plate MaxiSorp Surface C, Nunc
96well plate polypropylene for compound dilution in DMSO
CHO Tie2/DHFR (transfected cells)
PBS-; PBS++, DMSO
MEM alpha Medium with Glutamax-I without Ribonucleosides and
   Deoxyribonucleosides (Gibco #32561-029)
   with 10% FCS after dialysis! and 1% PenStrep
Lysis buffer: 1 Tablet "Complete" protease inhibitor
   1 cap Vanadate (1 mL > 40 mg/mL; working solution 2 mM)
   ad 50 mL with Duschl-Puffer
   pH 7.6
Anti-Tie2-antibody 1 : 425 in Coating Buffer pH 9.6
   Stock solution: 1.275 mg/mL > working.: 3 µg/mL
PBST: 2 bottles PBS(10x) + 10ml Tween, fill up with VE-water
RotiBlock 1 : 10 in VE-water
Anti-Phosphotyrosine HRP-Conjugated 1 : 10000 in 3% TopBlock
   3% TopBlock in PBST
BM Chemiluminescence ELISA Substrate (POD)
   solution B 1 : 100 solution A
SF9 cell culture medium
Ang2-Fc in SF9 cell culture medium

### Cell experiment:

Dispense 5 x 10⁴ cells / well / 98 µL in 96well tissue culture plate
Incubate at 37 °C / 5% CO₂
After 24 h add compounds according to desired concentrations
Add also to control and stimulated values without compounds 2 µL DMSO
And mix for a few min at room temperature
Add 100 µL Ang2-Fc to all wells except control, which receives insect medium
Incubate 20 min at 37 °C.
Wash 3x with PBS++
Add 100 µl Lysis buffer / well and shake a couple of min at room temperature
Store lysates at 20 °C before utilizing for the ELISA

### Performance of sandwich-ELISA

Coat 96well FluoroNunc Plate MaxiSorp Surface C with anti-Tie2 mAb
1 : 425 in Coating buffer pH 9.6; 100 µL / well overnight at 4 °C
Wash 2x with PBST
Block plates with 250 µL / well RotiBlock 1 : 10 in VE-water
Incubate for 2 h at room temperature or overnight at 4 °C shaking
Wash 2x in PBST
Add thawed lysates to wells and incubate overnight shaking at 4 °C
Wash 2x with PBST
Add 100 µL / well anti-Phosphotyrosine HRP-Conjugated 1 : 10000 in 3%
TopBlock (3% TopBlock in PBST) and incubate overnight under shaking
Wash 6x with PBST
Add 100 µL / well BM Chemiluminescence ELISA Substrate (POD)
solutions 1 und 2 (1 : 100)
Determine luminescence with the LumiCount.

### Assay 2: Tie-2-Kinase HTRF-Assay without kinase preactivation

Tie2-inhibitory activity of compounds of the present invention was quantified employing two Tie2 HTRF assay as described in the following paragraphs.

A recombinant fusion protein of GST and the intracellular domains of Tie-2, expressed in insect cells (Hi-5) and purified by Glutathion-Sepharose affinity chromatography was used as kinase. Alternatively, commercially available GST-Tie2-fusion protein (Upstate Biotechnology, Dundee, Scotland) can be used As substrate for the kinase reaction the biotinylated peptide biotin-Ahx-EPKDDAYPLYSDFG (C-terminus in amid form) was used which can be purchased e.g. from the company Biosynthan GmbH (Berlin-Buch, Germany). Detection of phosphorylated product is achieved specifically by a trimeric detection complex consisting of the phosphorylated substrate, streptavidin-XLent (SA-XLent) which binds to biotin, and Europium Cryptate-labeled anti-phosphotyrosine antibody PT66 which binds to phosphorylated tyrosine.

Tie-2 (3.5 ng/measurement point) was incubated for 60 min at 22°C in the presence of 10 µM adenosine-tri-phosphate (ATP) and 1 µM substrate peptide (biotin-Ahx-EPKDDAYPLYSDFG-NH₂) with different concentrations of test compounds (0 µM and concentrations in the range 0.001 - 20 µM) in 5 µl assay buffer [50 mM Hepes/NaOH pH 7, 10 mM MgCl₂, 0.5 mM MnCl₂, 1.0 mM dithiothreitol, 0.01% NP40, protease inhibitor mixture ("Complete w/o EDTA" from Roche, 1 tablet per 2.5 ml), 1 % (v/v) dimethylsulfoxide]. The reaction was stopped by the addition of 5 µl of an aqueous buffer (25 mM Hepes/NaOH pH 7.5, 0.28% (w/v) bovine serum albumin) containing EDTA (90 mM) and the HTRF (Homogeneous Time Resolved Fluorescence) detection reagents streptavidine-XLent (0.2 µM, from Cis Biointernational, Marcoule, France) and PT66-Eu-Chelate (0.3 ng/µl; a europium-chelate labelled anti-phospho-tyrosine antibody from Perkin Elmer).

The resulting mixture was incubated 1 h at 22°C to allow the binding of the biotinylated phosphorylated peptide to the streptavidine-XLent and the PT66-Eu-Chelate. Subsequently the amount of phosphorylated substrate peptide was evaluated by measurement of the resonance energy transfer from the PT66-Eu-Chelate to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate peptide. The data were normalised (enzyme reaction without inhibitor = 0 % inhibition, all other assay components but no enzyme = 100 % inhibition) and IC₅₀ values were calculated by a 4 parameter fit using an inhouse software.

### Assay 3: Tie-2-Kinase HTRF-Assay with kinase preactivation

A recombinant fusion protein of GST and the intracellular domains of Tie-2, expressed in insect cells (Hi-5) and purified by Glutathion-Sepharose affinity chromatography was used as kinase. As substrate for the kinase reaction the biotinylated peptide biotin-Ahx-EPKDDAYPLYSDFG (C-terminus in amid form) was used which can be purchased e.g. from the company Biosynthan GmbH (Berlin-Buch, Germany).

For activation, Tie-2 was incubated at a conc. 12.5 ng/µl of for 20 min at 22°C in the presence of 250 µM adenosine-tri-phosphate (ATP) in assay buffer [50 mM Hepes/NaOH pH 7, 10 mM MgCl₂, 0.5 mM MnCl₂, 1.0 mM dithiothreitol, 0.01% NP40, protease inhibitor mixture ("Complete w/o EDTA" from Roche, 1 tablet per 2.5 ml)].

For the subsequent kinase reaction, the preactivated Tie-2 (0.5 ng/measurement point) was incubated for 20 min at 22°C in the presence of 10 µM adenosine-tri-phosphate (ATP) and 1 µM substrate peptide (biotin-Ahx-EPKDDAYPLYSDFG-NH₂) with different concentrations of test compounds (0 µM and concentrations in the range 0.001 - 20 µM) in 5 µl assay buffer [50 mM Hepes/NaOH pH 7, 10 mM MgCl₂, 0.5 mM MnCl₂, 0.1 mM sodium ortho-vanadate, 1.0 mM dithiothreitol, 0.01% NP40, protease inhibitor mixture ("Complete w/o EDTA" from Roche, 1 tablet per 2.5 ml), 1 % (v/v) dimethylsulfoxide]. The reaction was stopped by the addition of 5 µl of an aqueous buffer (25 mM Hepes/NaOH pH 7.5, 0.28% (w/v) bovine serum albumin) containing EDTA (90 mM) and the HTRF (Homogeneous Time Resolved Fluorescence) detection reagents streptavidine-XLent (0.2 µM, from Cis Biointernational, Marcoule, France) and PT66-Eu-Chelate (0.3 ng/µl; a europium-chelate labelled anti-phospho-tyrosine antibody from Perkin Elmer).

The resulting mixture was incubated 1 h at 22°C to allow the binding of the biotinylated phosphorylated peptide to the streptavidine-XLent and the PT66-Eu-Chelate. Subsequently the amount of phosphorylated substrate peptide was evaluated by measurement of the resonance energy transfer from the PT66-Eu-Chelate to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate peptide. The data were normalised (enzyme reaction without inhibitor = 0 % inhibition, all other assay components but no enzyme = 100 % inhibition) and IC₅₀ values were calculated by a 4 parameter fit using an inhouse software.

### Assay 4: CDK2 HTRF Assay

CDK2/CycE -inhibitory activity of compounds of the present invention was quantified employing the CDK2/CycE HTRF assay as described in the following paragraphs.

Recombinant fusion proteins of GST and human CDK2 and of GST and human CycE, expressed in insect cells (Sf9) and purified by Glutathion-Sepharose affinity chromatography, were purchase from ProQinase GmbH (Freiburg, Germany). As substrate for the kinase reaction biotinylated peptide biotin-Ttds-YISPLKSPYKISEG (C-terminus in amid form) was used which can be purchased e.g. form the company JERINI peptide technologies (Berlin, Germany).
CDK2/CycE was incubated for 60 min at 22°C in the presence of different concentrations of test compounds in 5 µl assay buffer [50 mM Tris/HCl pH 8.0, 10 mM MgCl2, 1.0 mM dithiothreitol, 0.1 mM sodium ortho-vanadate, 10 µM adenosine-tri-phosphate (ATP), 0.75 µM substrate, 0.01% (v/v) Nonidet-P40 (Sigma), 1 % (v/v) dimethylsulfoxide]. The concentration of CDK2/CycE was adjusted depending of the activity of the enzyme lot and was chosen appropriate to have the assay in the linear range, typical concentrations were in the range of 1ng/ml. The reaction was stopped by the addition of 5 µl of a solution of HTRF detection reagents (0.2 µM streptavidine-XLent and 3.4 nM Phospho-(Ser) CDKs Substrate Antibody [product #2324B, Cell Signalling Technology, Danvers, MA, USA} and 4 nM Prot-A-EuK [Protein A labeled with Europium Cryptate from Cis biointernational, France, product no. 61PRAKLB]) in an aqueous EDTA-solution (100 mM EDTA, 800 mM KF, 0.2 % (w/v) bovine serum albumin in 100 mM HEPES/NaOH pH 7.0).

### Assay 5: KDR kinase assay

KDR inhibitory activity of compounds of the present invention was quantified employing the KDR HTRF assay as described in the following paragraphs.

GST-tagged recombinant kinase domain of the human KDR expressed in SF-9 cells was used as kinase. As substrate for the kinase reaction the biotinylated peptide biotin-Ahx-DFGLARDMYDKEYYSVG (C-terminus in acid form) was used which can be purchased e.g. form the company Biosynthan GmbH (Berlin-Buch, Germany).
KDR was incubated for 45 min at 22°C in the presence of different concentrations of test compounds in 5 µl assay buffer [50 mM Hepes/NaOH pH 7.0, 25 mM MgCl₂, 5 mM MnCl₂, 1.0 mM dithiothreitol, 0.1 mM sodium ortho-vanadate, 10 µM adenosine-tri-phosphate (ATP), 0.5 µM substrate, 0.001% (v/v) Nonidet-P40 (Sigma), 1 % (v/v) dimethylsulfoxide]. The concentration of KDR was adjusted depending of the activity of the enzyme lot and was chosen appropriate to have the assay in the linear range. The reaction was stopped by the addition of 5 µl of a solution of HTRF detection reagents (0.1 µM streptavidine-XLent and 2 nM PT66-Eu-Chelate, an europium-chelate labelled anti-phospho-tyrosine antibody from Perkin Elmer) in an aqueous EDTA-solution (125 mM EDTA, 0.2 % (w/v) bovine serum albumin in 50 mM HEPES/NaOH pH 7.0).
The resulting mixture was incubated 1 h at 22°C to allow the binding of the biotinylated phosphorylated peptide to the streptavidine-XLent and the PT66-Eu-Chelate. Subsequently the amount of phosphorylated substrate was evaluated by measurement of the resonance energy transfer from the PT66-Eu-Chelate to the streptavidine-XLent. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition) and IC50 values were calculated by a 4 parameter fit using an inhouse software.

### Assay 6: Plk1 inhibition assay

### 1. Principle (SPA)

Plk1 phosphorylates serine/threonine residues of the biotinylated substrate bovine □-casein in the presence of [□-³³P]ATP. Detection of the radiolabeled phosphorylated product is achieved by binding to streptavidine-coated SPA beads. The biotin moieties of biotinylated casein bind with high affinity to the streptavidine. The radiolabeled biotinylated casein produced by the Plk1 kinase reaction is able to generate a chemoluminescent signal when strepatavidine-mediated binding occurs to the scintillant-containing surface of the beads due to the close proximity of the radiolabel and the scintillant. Unphosphorylated substrate does not give rise to such a signal because it does not contain radiolabeled phosphate groups. Any free [□-³³P]ATP which remains unbound in solution does not significantly contribute to the background signal obtained due to its non-proximity. The signals obtained are therefore indicative of the Plk1 kinase activity. Measurement is performed in a Perkin-Elmer TopCount instrument.

### 2. Material

Enzyme: Purified human recombinant PLK1 [N-terminally 6His-tagged full length Plk1], prepared in house, conc.: 0.4 µg/µl
Substrate: biotinylated bovine □-casein. Unbiotinylated casein obtained from Sigma was biotinylated by standard procedures using N-hydroxysuccinimide (NHS) ester of biotin.
SPA-beads: PVT streptavidin SPA Beads, Amersham (# RPNQ0007)
Reference compound: Wortmannin
Assay plates: 384-well plates, small volume, white, Greiner (# 784075)
Buffer Materials: Hepes: Sigma (# H-4034), MnCl2: Sigma (# M-1787), MgCl2: Sigma [# M-1028], NaCL Merck [#1.06404.100], DTT: Biomol (# 04010), NP40: Fluka (# 74385), Complete EDTA-free: Roche [# 1873580], ATP: Amersham (# 27-2056), 33P-gamma-ATP: Perkin Elmer (Easytides # NEG/602H), EDTA: Sigma (# E-7889), PBS: Gibco (#14190-094), ATP for stopping solution.: Sigma #(A-7699), DMSO: Merck [# 8.02912.1000]
Sealing tape: Greiner (#676080)
Reader: TopCount instrument (PerkinElmer)
Working Reagents:
   Assay buffer: 2.5mM MnCl2, 5mM MgCl2, 120mM NaCl, 1 mM DTT, 50mM Hepes pH 7.0, 0.01% NP40, 1 x Complete (protease inhibitor cocktail)
   Enzyme working solution: 2.86ng/µl PLK1 in assay buffer
   Substrate working solution: 1µM ATP, 1.4µM biotinylated a-Casein, 2.0nCi/µl in assay buffer
   Stop/Detection solution: 50µM ATP, 20mM EDTA, 5µg/µl beads in PBS

### 3. Assay protocol for small volume screen (all steps at 20°C, pipetted with CyBi-Well pipettor)

1. 0.75µl Compound in 30% or 100% DMSO
2. addition of 3.5 µl Enzyme working solution
3. addition of 3.5 µl Substrate working solution incubation 180 min
4. addition of 10 µl Stop/Detection solution
5. sealing of the plates, incubation 30 min, 10 min centrifugation at 1500rpm
6. Measurement in TopCount reader (60 sec/well)

Final concentrations, calculated for 7 µl reaction volume: PLK1 10ng/well; a-Casein 0.7 µM; ATP 0.5 µM; gamma-33P-ATP 7nCi/well, 2.5mM MnCl2, 5mM MgCl2, 120mM NaCl, 1 mM DTT, 50mM Hepes pH 7.0, 0.01% NP40, 1 x Complete, 3% or 10% DMSO

The data were normalised (enzyme reaction without inhibitor = 0 % inhibition, enzyme reaction in the presence of 3.0E-06 M wortmannin= 100 % inhibition) and IC₅₀ values were calculated by a 4-parameter fit using an in house software.

Compounds of the present invention were found to possess potent activity as inhibitors of the angiogenesis-relevant kinases Tie2 and KDR (= VEGFR2). Compounds of the present invention inhibit Tie2 kinase activity and KDR kinase activity with IC₅₀ values below 1 □M, more preferred compounds inhibit Tie2 kinase activity with IC₅₀ values below 0.25 □M and even more preferred compounds inhibit Tie2 kinase activity and KDR kinase activity with IC₅₀ values below 0.25 □M. Surprisingly compounds of the present invention were found to possess a selectivity profile, which is highly advantageous for compounds to be used as purely antiangiogenic agents, as they are more potently inhibiting Tie2 and KDR activity than inhibiting the cell cycle kinase CDK2 and Plk1. Compounds of the present invention inhibit CDK2 enzymatic activity with IC₅₀ values greater than 1 □M, more preferred compounds inhibit CDK2 enzymatic activity with IC₅₀ values greater than 10 □M. Compounds of the present invention inhibit Plk1 activity with IC₅₀ values greater than 10 □M.

Selected data are given in the following table. The IC₅₀ values were converted to pIC₅₀ values, i.e. -log IC₅₀ in molar concentration.

| **Example No.** | **Tie 2 activity (assay 2)** | **KDR activity (assay 5)** | **CDK2 activity (assay 4)** | **Plk 1 activity (assay 6)** |
|---|---|---|---|---|
| 4 | ++ | ++ | -- | -- |
| 5 | ++ | + | -- | -- |
| 6 | + | ++ | - | -- |
| 8 | + | ++ | - | -- |
| 13 | ++ | ++ | -- | -- |

| | | | | |
|---|---|---|---|---|
| -- stands for plC₅₀ < 5.0 - stands for plC₅₀ 5.0 - 6.0 + stands for plC₅₀ 6.0 - 7.0 ++ stands for plC₅₀ ≥ 7.0. | | | | |

## Claims

1. A compound of general formula (I) : 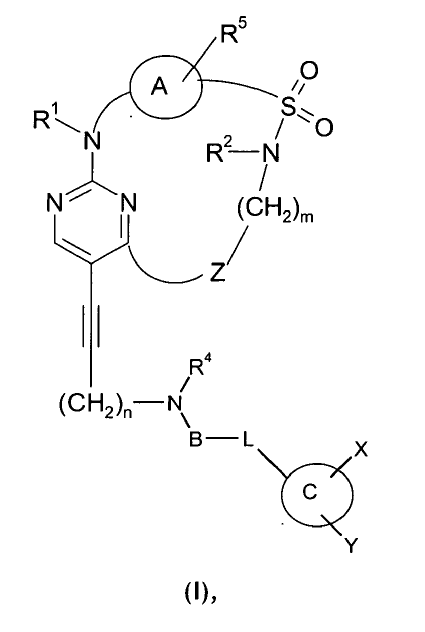 wherein
A is phenylene or C₆-heteroarylene;
C is arylene or heteroarylene ;
Z is selected from the group comprising, preferably consisting of, O, S, NR³ and CHR³ ;
R¹, R², R³, R⁴ and R⁶ independently from each other are selected from the group comprising, preferably consisting of, hydrogen and C₁-C₆-alkyl, wherein C₁-C₆-alkyl is unsubstituted or singly or multiply substituted with -OR⁷ or -NR⁷R⁸;
R⁵ is selected from the group comprising, preferably consisting of, hydrogen, halogen, nitro, cyano, C₁-C₆-alkyl, -(CH₂)ₚOR^{7a}, - (CH₂)ₚNR^{7a}R^{8a}, -(CH₂)ₚC(O)R^{7a}, -(CH₂)ₚNHC(O)R^{7a}, - (CH₂)ₚNHC(O)NR^{7a}R^{8a}, -(CH₂)ₚNHS(O)₂R^{7a}, and -(CH₂)ₚC(O)NR^{7a}R^{8a} ;
R⁷ , R^{7a} , R^{7b} , R^{7c},R^{7d}, R^{7e} , R⁸ R^{8a}, R^{8b}, R^{8c}, R^{8d}, and R^{8e} independently from each other represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7b}, -NR^{7b}R^{8b}, C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a} ; or
R⁷ and R⁸, R^{7a} and R^{8a}, R^{7b} and R^{8b}, R^{7c} and R^{8c}, and R^{7e} and R^{8e} together with the nitrogen atom to which they are attached in groups -NR⁷R⁸, -NR^{7a}R^{8a} ,-NR^{7b}R^{8b}, -NR^{7c}R^{8c}, and -NR^{7e}R^{8e} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
R⁹, R^{9a}, independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH2)_{q'}-heteroaryl, wherein said residues are unsubstituted or substituted one or more times independently from each other with halogen, nitro, cyano, C₁-C₆-alkyl, C₁-C₆-haloalkyl ; or
R⁹ and R^{9a}, together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)-, and -S(O)₂- group, and can optionally contain one or more double bonds ;
B is selected from the group comprising, preferably consisting of, -C(O)- , -C(S)-, -C(=NR⁶)-, -C(O)NR⁶-, -C(=NR⁶)NR⁶-, -S(O)₂-, -S(O)(=NR⁶)-, - S(=NR⁶)₂-, -C(S)NR⁶-, -C(O)C(O)-, -C(O)C(O)NR⁶-, -C(O)NR⁶C(O)-, - C(S)NR⁶C(O)-, -C(O)NR⁶C(S)-;
L represents a bond or is selected from the group comprising, preferably consisting of C₁-C₆-alkylene or C₃-C₁₀-cycloalkylene ;
X is selected from the group comprising, preferably consisting of, hydrogen, halogen, nitro, cyano, -(CH₂)ᵣOR^{7c}, -(CH₂)ᵣNR^{7c}R^{8c}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7c}, -(CH₂)ᵣC(O)NR^{7c}R^{8c} and - (CH₂)ᵣS(O)₂NR^{7c}R^{8c} ;
Y is selected from the group comprising, preferably consisting of, hydrogen, halogen, nitro, cyano, -(CH₂)ₛOR^{7e}, -(CH₂)ₛNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7e}, -(CH₂)ₛC(O)NR^{7e}R^{8e} and - (CH₂)ₛS(O)₂NR^{7e}R^{8e};
m represents an integer of 3, 4, 5, or 6 ;
n is an integer of 1 or 2 ; and
p, q, q', r, s independently from each other represent an integer of 0, 1, or 2.

2. The compound of general formula according to claim 1, wherein :
A is meta-phenylene ;
C is arylene or heteroarylene ;
Z is NR³;
R¹, R², R³ and R⁵ are hydrogen;
R⁴ and R⁶ independently from each other, are selected from the group comprising, preferably consisting of, hydrogen and C₁-C₆-alkyl ;
R^{7b}, R^{7c}, R^{7d}, R^{7e}, R^{8b}, R^{8c}, R^{8d}, and R^{8e} independently from each other represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocyctoalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7b}, -NR^{7b}R^{8b} , C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a}; or
R^{7b} and R⁸b, R^{7c} and R^{8c}, and R^{7e} and R^{8e} together with the nitrogen atom to which they are attached in groups -NR^{7b}R^{8b}, -NR^{7c}R^{8c}, and -NR^{7e}R^{8e} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
R⁹, R^{9a}, independently from each other represent hydrogen, or are selected from the group comprising, preferably consisting of, C₁-C₆-alkyl, C₁-C₆-alkoxy, C₂-C₆-alkenyL, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
R⁹ and R^{9a}, together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)-, and -S(O)₂- group, and can optionally contain one or more double bonds ;
B is selected from the group comprising, preferably consisting of, -C(O)- , -C(S)-, -C(=NR⁶)-, -C(O)NR⁶-, -C(=NR⁶)NR⁶-, -S(O)₂-, -S(O)(=NR⁶)-, - S(=NR⁶)₂-, -C(S)NR⁶-, -C(O)C(O)-, -C(O)C(O)NR⁶-, -C(O)NR⁶C(O)-, - C(S)NR⁶C(O)-, -C(O)NR⁶C(S)-;
L represents a bond or is selected from the group comprising, preferably consisting of C₁-C₆-alkylene or C₃-C₁₀-cycloalkylene ;
X is selected from the group comprising, preferably consisting of, hydrogen, halogen, nitro, cyano, -(CH₂)ᵣOR^{7c}, -(CH₂)ᵣNR^{7c}R^{8c}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7c}, -(CH₂)ᵣC(O)NR^{7c}R^{8c} and - (CH₂)ᵣS(O)₂NR^{7c}R^{8c} ;
Y is selected from the group comprising, preferably consisting of, hydrogen, halogen, nitro, cyano, -(CH₂)ₛOR^{7e}, -(CH₂)ₛNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7e}, -(CH₂)ₛC(O)NR^{7e}R^{8e} and - (CH₂)ₛS(O)₂NR^{7e}R^{8e} ;
m represents an integer of 3 ;
n is an integer of 1 or 2 ; and
q, q', r, s independently from each other represent an integer of 0, 1, or 2.

3. The compound of general formula according to claim 1 or 2, wherein :
A is meta-phenylene ;
C is arylene or heteroarylene ;
Z is NR³;
R¹, R², R³, and R⁵ are hydrogen;
R⁴ and R⁶ independently from each other, are selected from the group comprising, preferably consisting of, hydrogen and C₁-C₆-alkyl;
R^{7b}, R^{7c}, R^{7d}, R^{7e}, R^{8b}, R^{8c}, R^{8d}, and R^{8e} independently from each other represent hydrogen, -C(O)R⁹, -S(O)₂R⁹ or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q}-aryl and -(CH₂)_{q}-heteroaryl, wherein said residues are unsubstituted or substituted one or more times independently from each other with halogen, nitro, cyano, C₁-C₆-alkyl, -OR^{7b}, -NR^{7b}R^{8b} , C₁-C₆-haloalkyl, C₁-C₆-haloalkoxy, C₁-C₆-alkylthio, -C(O)OR⁹, -C(O)NR⁹R^{9a}, and -S(O)₂NR⁹R^{9a}; or
R^{7b} and R^{8b}, R^{7c} and R^{8c}, and R^{7e} and R^{8e} together with the nitrogen atom to which they are attached in groups -NR^{7b}R^{8b}, -NR^{7c}R^{8c}, and -NR^{7e}R^{8e} form a 3 to 10 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
R⁹, R^{9a}, independently from each other represent hydrogen, or are selected from the group comprising, preferably consisting of, C₁-C₆-alkyl, C₁-C₆-alkoxy, C₂-C₆-alkenyl, C₂-C₆-alkynyl, C₃-C₁₀-cycloalkyl, C₃-C₁₀-heterocycloalkyl, -(CH₂)_{q'}-aryl and -(CH₂)_{q'}-heteroaryl, wherein said residues are unsubstituted or substituted one or more times independently from each other with halogen, nitro, cyano, C₁-C₆-alkyl, C₁-C₆-haloalkyl ; or
R⁹ and R^{9a}, together with the nitrogen atom to which they are attached form a 3 to 10 membered heterocycloalkyl ring, wherein the carbon backbone of this heterocyoloalkyl ring can optionally be interrupted one or more times, the same way or differently, by a member of the group comprising, preferably consisting of, NH, NR^{7d}, oxygen or sulfur, and can optionally be interrupted one or more times, the same way or differently, with a -C(O)-, -S(O)- , and -S(O)₂- group, and can optionally contain one or more double bonds ;
B is selected from the group comprising, preferably consisting of, -C(O)- , -C(O)NR⁶-, -S(O)₂- ;
L represents a bond or is selected from the group comprising, preferably consisting of C₁-C₆-alkylene or C₃-C₁₀-cycloalkylene ;
X is selected from the group comprising, preferably consisting of, hydrogen, halogen, nitro, cyano, -(CH₂)ᵣOR^{7c}, -(CH₂)ᵣNR^{7c}R^{8c}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ᵣC(O)R^{7c}, -(CH₂)ᵣC(O)NR^{7c}R^{8c} and - (CH₂)ᵣS(O)₂NR^{7c}R^{8c} ;
Y is selected from the group comprising, preferably consisting of, hydrogen, halogen, nitro, cyano, -(CH₂)ₛOR^{7e}, -(CH₂)ₛNR^{7e}R^{8e}, C₁-C₆-alkyl, C₃-C₁₀-cycloalkyl, C₃-C₁₀-heterocycloalkyl, C₁-C₆-haloalkyl, C₁-C₆-haloalkoxy, C₁-C₆-alkylthio, -(CH₂)ₛC(O)R^{7e}, -(CH₂)ₛC(O)NR^{7e}R^{8e} and - (CH₂)ₛS(O)₂NR^{7e}R^{8e} ;
m represents an integer of 3 ;
n is an integer of 1 or 2 ; and
q, q', r, s independently from each other represent an integer of 0, 1, or 2.

4. The compound of general formula according to any one of claims 1 to 3, wherein :
A is meta-phenylene ;
C is phenylene ;
Z is NR³ ;
R¹,R²,R³, R⁴, R⁵ and R⁶ are hydrogen ;
R^{7b}, R^{7c}, R^{7d}, R^{7e}, R^{8b}, R^{8c}, independently from each other represent hydrogen, or C₁-C₆-alkyl, wherein said C₁-C₆-alkyl is unsubstituted or substituted one or more times independently from each other with -OR^{7b}, -NR^{7b}R^{8b} ; or
R^{7b} and R^{8b}, R^{7c} and R^{8c}, together with the nitrogen atom to which they are attached in groups -NR^{7b}R^{8b}, and -NR^{7c}R^{8c}, form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen ;
B is -C(O)NR⁶-, or -S(O)₂- ;
L represents a bond or is C₁-alkylene ;
X is selected from the group comprising, preferably consisting of, hydrogen, halogen, -(CH₂)ᵣOR^{7c}, -(CH₂)ᵣNR^{7c}R^{8c}, C₁-C₆-alkyl, C₁-C₆-haloalkyl, C₁-C₆-haloalkoxy ;
Y is selected from the group comprising, preferably consisting of, hydrogen, halogen, -(CH₂)ₛOR^{7e}, C₁-C₆-alkyl, C₁-C₆-haloalkyl ;
m represents an integer of 3 ;
n is an integer of 1 or 2 ; and
r represents an integer of 0 or 1 ; and
s is an integer of 0.

5. The compound of general formula according to any one of claims 1 to 4, wherein :
A is meta-phenylene ;
C is phenylene ;
Z is NR³ ;
R¹, R², R³, R⁴, R⁵ and R⁶ are hydrogen ;
R^{7b}, R^{7c}, R^{7d}, R^{7e}, R^{8b}, R^{8c}, independently from each other represent hydrogen, or C₁-C₆-alkyl, wherein said C₁-C₆-alkyl is unsubstituted or substituted one or more times independently from each other with -OR^{7b}, -NR^{7b}R^{8b}; or
R^{7b} and R^{8b}, R^{7c} and R^{8c}, together with the nitrogen atom to which they are attached in groups -NR^{7b}R^{8b}, and -NR^{7c}R^{8c}, form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen ;
B is -C(O)NR⁶- ;
L represents a bond or is C₁-alkylene ;
X is selected from the group comprising, preferably consisting of, hydrogen, halogen, -(CH₂)ᵣOR^{7c}, -(CH₂)ᵣNR^{7c}R^{8c}, C₁-C₆-alkyl, C₁-C₆-haloalkyl, C₁-C₆-haloalkoxy ;
Y is selected from the group comprising, preferably consisting of, hydrogen, halogen, -(CH₂)ₛOR^{7e}, C₁-C₆-alkyl, C₁-C₆-haloalkyl ;
m represents an integer of 3 ;
n is an integer of 1 or 2 ; and
r represents an integer of 0 or 1 ; and
s is an integer of 0.

6. The compound of general formula according to any one of claims 1 to 4, wherein :
A is meta-phenylene ;
C is phenylene ;
Z is NR³ ;
R¹, R², R³, R⁴, R⁵ and R⁶ are hydrogen ;
R^{7b}, R^{7c}, R^{7d}, R^{7e}, R^{8b}, R^{8c} independently from each other represent hydrogen, or C₁-C₆-alkyl, wherein said C₁-C₆-alkyl is unsubstituted or substituted one or more times independently from each other with -OR^{7b}, -NR^{7b}R^{8b}; or
R^{7b} and R^{8b}, R^{7c} and R^{8c}, together with the nitrogen atom to which they are attached in groups -NR^{7b}R^{8b}, and -NR^{7c}R^{8c}, form a 3 to 6 membered heterocycloalkyl ring, wherein the carbon backbone of this heterocycloalkyl ring can optionally be interrupted one or more times, the same way or differently, by a member of the group comprising, preferably consisting of, NH, NR^{7d}, oxygen;
B is -S(O)₂- ;
L represents a bond or is C₁-alkylene ;
X is selected from the group comprising, preferably consisting of, hydrogen, halogen, (CH₂)ᵣOR^{7c}, -(CH₂)ᵣNR^{7c}R^{8c}, C₁-C₆-alkyl, C₁-C₆-haloalkyl, C₁-C₆-haloalkoxy ;
Y is selected from the group comprising, preferably consisting of, hydrogen, halogen, -(CH₂)ₛOR^{7e}, C₁-C₆-alkyl, C₁-C₆-haloalkyl
m represents an integer of 3 ;
n is an integer of 1 or 2 ; and
r represents an integer of 0 or 1 ; and
s is an integer of 0.

7. The compound of general formula (I) according to any one of claims 1 to 6, which is selected from the group consisting of :
1-[3-(13,13-Dioxo-13λ⁶-thia-2,4,8,12,19-pentaaza-tricyclo[12.3.1.1^{3,7}]-nonadeca-1(18),3(19),4,6,14,16-hexaen-6-yl)-prop-2-ynyl]-3-phenyl-urea ;
1-[3-(13,13-Dioxo-13λ⁶-thia-2,4,8,12,19-pentaaza-tricyclo[12.3.1.1^{3,7}]-nonadeca-1(18),3(19),4,6,14,16-hexaen-6-yl)-prop-2-ynyl]-3-p-tolyl-urea ;
1-[3-(13,13-Dioxo-13λ⁶-thia-2,4,8,12,19-pentaaza-tricyclo[12.3.1.1^{3,7}]-nonadeca-1(18),3(19),4,6,14,16-hexaen-6-yl)-prop-2-ynyl]-3-m-tolyl-urea ;
1-[3-(13,13-Dioxo-13λ⁶-thia-2,4,8,12,19-pentaaza-tricyclo[12.3.1.1^{3,7}]-nonadeca-1(18),3(19),4,6,14,16-hexaen-6-yl)-prop-2-ynyl]-3-(3-trifluoromethyl-phenyl)-urea ;
1-Benzyl-3-[3-(13, 13-dioxo-13λ⁶-thia-2,4,8,12,19-pentaaza-tricyclo[12.3.1.1^{3,7}]-nonadeca-1(18),3(19),4,6,14,16-hexaen-6-yl)-prop-2-ynyl]-urea ;
1-[4-(13,13-Dioxo-13λ⁶-thia-2,4,8,12,19-pentaaza-tricyclo[12.3.1.1^{3,7}]-nonadeca-1(18),3(19),4,6,14,16-hexaen-6-yl)-but-3-ynyl]-3-phenyl-urea ;
1-[4-(13,13-Dioxo-13λ⁶-thia-2,4,8,12,19-pentaaza-tricyclo[12.3.1.1^{3,7}]-nonadeca-1(18),3(19),4,6,14,16-hexaen-6-yl)-but-3-ynyl]-3-(3-trifluoromethyl-phenyl)-urea ;
2,3-Dichloro-N-[3-(13,13-dioxo-13λ⁶-thia-2,4,8,12,19-pentaaza-tricyclo[12.3.1.1^{3,7}]-nonadeca-1 (18),3(19),4,6,14,16-hexaen-6-yl)-prop-2-ynyl]-benzene-sulfonamide ;
N-[4-(13,13-Dioxo-13λ⁶-thia-2,4,8,12,19-pentaaza-tricyclo[12.3.1.1^{3,7}]-nonadeca-1(18),3(19),4,6,14,16-hexaen-6-yl)-but-3-ynyl]-benzene-sulfonamide ;
2,3-Dichloro-N-[4-(13,13-dioxo-13λ⁶-thia-2,4,8,12,19-pentaaza-tricyclo[12.3.1.1^{3,7}]-nonadeca-1(18),3(19),4,6,14,16-hexaen-6-yl)-but-3-ynyl]-benzene-sulfonamide ;
N-[4- (13, 13-Dioxo- 13λ⁶-thia-2,4,8,12,19-pentaaza-tricyclo[12.3.1.1^{3,7}]-nonadeca-1 (18),3(19),4,6,14,16-hexaen-6-yl)-but-3-ynyl]-3-fluoro-benzene-sulfonamide ;
N-[4-(13,13-Dioxo-13λ⁶-thia-2,4,8,12,19-pentaaza-tricyclo[12.3.1.1^{3,7}]-nonadeca-1(18),3(19),4,6,14,16-hexaen-6-yl)-but-3-ynyl]-3-trifluoromethyl-benzene-sulfonamide ;
N-[4-(13,13-Dioxo-13λ⁶-thia-2,4,8,12,19-pentaaza-tricyclo[12.3.1.1^{3,7}]-nonadeca-1 (18),3(19),4,6,14,16-hexaen-6-yl)-but-3-ynyl]-2-fluoro-5-methyl-benzene-sulfonamide ;
N-[4-(13,13-Dioxo-13λ⁶-thia-2,4,8,12,19-pentaaza-tricyclo[12.3.1.1^{3,7}]-nonadeca-1 (18),3(19),4,6,14,16-hexaen-6-yl)-but-3-ynyl]-C-phenyl-methane-sulfonamide ;
1-Phenyl-cyclopropanecarboxylic acid [3-(13,13-dioxo-13λ⁶-thia-2,4,8,12,19-pentaaza-tricyclo[12.3.1.1^{3,7}]-nonadeca-1(18), 3(19),4,6,14,16-hexaen-6-yl)-prop-2-ynyl]-amide;
1-Phenyl-cyclopropanecarboxylic acid [4-(13, 13-dioxo-13λ⁶-thia-2,4,8,12,19-pentaaza-tricyclo[12.3.1.1^{3,7}]nonadeca-1 (18),3(19),4,6,14,16-hexaen-6-yl)-but-3-ynyl]-amide.

8. A method of preparing a compound of general formula (I) according to any one of claims 1 to 7, said method comprising the step of allowing an intermediate compound of general formula A : 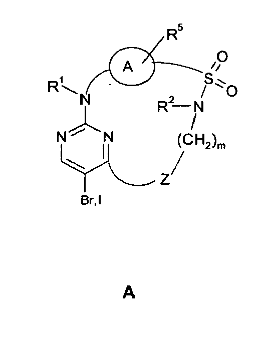 in which the meanings of R¹, R², R⁵, A, Z, and m are as defined in claim 1,
to react with an intermediate compound of general formula B : 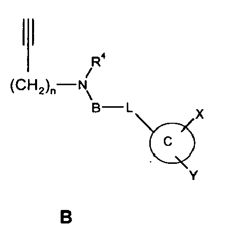 in which R⁴, B, C, L, X, Y, and n are as defined in claim 1,
thus providing a compound of general formula (I) : 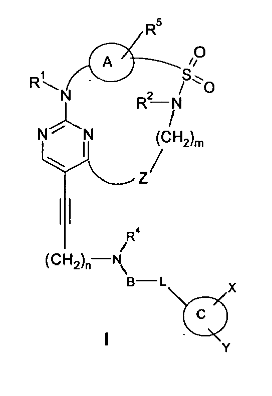 in which R¹, R², R⁴, R⁵, A, B, C, L, X, Y, Z, n, and m are as defined in claim 1.

9. A pharmaceutical composition which comprises a compound of general formula (I) according to any one of claims 1 to 7, or a pharmaceutically acceptable salt or an *in vivo* hydrolysable ester thereof, and a pharmaceutically-acceptable diluent or carrier.

10. Use of a compound of any one of claims 1 to 7 for manufacturing a pharmaceutical composition for the treatment of diseases of dysregulated vascular growth or of diseases which are accompanied with dysregulated vascular growth.

11. Use according to claim 10, wherein said diseases are tumours and/or metastases thereof.

12. Use according to claim 10, wherein said diseases are retinopathy, other angiogenesis dependent diseases of the eye, rheumatoid arthritis, and other inflammatory diseases associated with angiogenesis.

13. Use of claim 12, wherein said angiogenesis dependent diseases of the eye are cornea transplant rejection, age-related macular degeneration.

14. Use according to claim 10, wherein said diseases are coronary and peripheral artery disease.

15. Use of claim 12, wherein said inflammatory diseases associated with angiogenesis are psoriasis, delayed type hypersensitivity, contact dermatitis, asthma, multiple sclerosis, restenosis, pulmonary hypertension, stroke, and diseases of the bowel.

16. Use according to claim 10, wherein said diseases are ascites, oedema such as brain tumour associated oedema, high altitude trauma, hypoxia induced cerebral oedema, pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

17. A method of treating a disease of dysregulated vascular growth or diseases which are accompanied with dysregulated vascular growth by administering an effective amount of a compound of general formula (I) according to any one of claims 1 to 7.

18. The method according to claim 17, wherein said diseases are tumours and/or metastases thereof.

19. The method according to claim 17, wherein said diseases are retinopathy, other angiogenesis dependent diseases of the eye, rheumatoid arthritis, and other inflammatory diseases associated with angiogenesis.

20. The method according to claim 19, wherein said angiogenesis dependent diseases of the eye are cornea transplant rejection, age-related macular degeneration.

21. The method according to claim 19, wherein said diseases are coronary and peripheral artery disease.

22. The method according to claim 19 wherein said inflammatory diseases associated with angiogenesis are psoriasis, delayed type hypersensitivity, contact dermatitis, asthma, multiple sclerosis, restenosis, pulmonary hypertension, stroke, and diseases of the bowel.

23. The method according to claim 17, wherein said diseases are ascites, oedema such as brain tumour associated oedema, high altitude trauma, hypoxia induced cerebral oedema, pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation during regeneration of damaged nerves, endometriosis, pre-eclampsia, postmenopausal bleeding and ovarian hyperstimulation.

24. Use of a compound of general formula A : 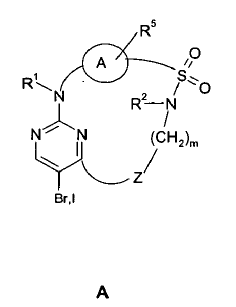 in which the meanings of R¹, R², R⁵, A, Z, and m are as defined in claim 1,
for the preparation of a compound of general formula (I) : 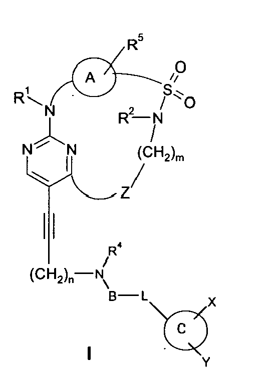 in which R¹, R², R⁴, R⁵, A, B, C, L, X, Y, Z, n, and m are as defined in claim 1.

25. Use of a compound of general formula B : 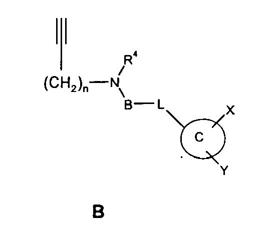 in which R⁴, B, C, L, X, Y, and n are as defined in claim 1,
for the preparation of a compound of general formula (I) : 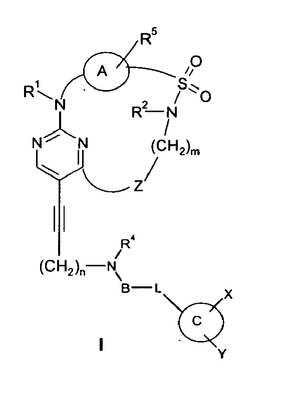 in which R¹, R², R⁴, R⁵, A, B, C, L, X, Y, Z, n, and m are as defined in claim 1.
